# EUROPEAN PATENT SPECIFICATION

(11) **EP 3 322 420 B1**
(45) Date of publication and mention of the grant of the patent: **29.12.2021**
(21) Application number: 16824927.4
(22) Date of filing: 08.07.2016
(51) Int. Cl.: A61K 31/4439, C07D 498/04

(54) **BICYCLIC HETEROCYCLES AS INHIBITORS OF CHOLESTEROL ESTER TRANSFER PROTEIN**
BICYCLISCHE HETEROCYCLEN ALS INHIBITOREN DES CHOLESTERINESTERTRANSFERPROTEINS
HÉTÉROCYCLES BICYCLIQUES EN TANT QU'INHIBITEURS DE LA PROTÉINE DE TRANSFERT DES ESTERS DE CHOLESTÉROL

(30) Priority: 13.07.2015 US 201562191735 P
(43) Date of publication of application: 23.05.2018
(73) Proprietor: Merck Sharp & Dohme Corp., Rahway, NJ 07065-0907 (US)
(72) Inventor: SHAO, Pengcheng Patrick, Kenilworth, New Jersey 07033 (US); KATIPALLY, Revathi Reddy, Kenilworth, New Jersey 07033 (US); VACHAL, Petr, Rahway, New Jersey 07065-0907 (US); THATAI, Jayanth Thiruvellore, Bangalore 560 099 (IN); SARKAR, Sujit Kumar, Bangalore 560 099 (IN)
(74) Representative: Böhles, Elena
(86) International application number: PCT/US2016/041419
(87) International publication number: WO 2017/011279

(56) References cited:
- WO-A2-2004/072046
- WO-A2-2015/017302
- US-A1- 2014 378 493

## Description

### FIELD OF THE INVENTION

This invention relates to chemical compounds that inhibit cholesterol ester transfer protein (CETP) and that are expected to have utility in raising HDL-C, lowering LDL-C, and in the treatment and prevention of atherosclerosis.

### BACKGROUND OF THE INVENTION

Atherosclerosis and its clinical consequences, including coronary heart disease (CHD), stroke and peripheral vascular disease, represent a truly enormous burden to the health care systems of the industrialized world. In the United States alone, approximately 13 million patients have been diagnosed with CHD, and greater than one half million deaths are attributed to CHD each year. Further, this toll is expected to grow over the next quarter century as an epidemic in obesity and diabetes continues to grow.

It has long been recognized that in mammals, variations in circulating lipoprotein profiles correlate with the risk of atherosclerosis and CHD. The clinical success of HMG-CoA reductase inhibitors, especially the statins, in reducing coronary events is based on the reduction of circulating low density lipoprotein cholesterol (LDL-C), levels of which correlate directly with an increased risk for atherosclerosis. More recently, epidemiologic studies have demonstrated an inverse relationship between high density lipoprotein cholesterol (HDL-C) levels and atherosclerosis, leading to the conclusion that low serum HDL-C levels are associated with an increased risk for CHD

Metabolic control of lipoprotein levels is a complex and dynamic process involving many factors. One important metabolic control in man is the cholesteryl ester transfer protein (CETP), a plasma glycoprotein that catalyzes the movement of cholesteryl esters from HDL to the apoB containing lipoproteins, especially VLDL (see Hesler, C.B., et. al. (1987) Purification and characterization of human plasma cholesteryl ester transfer protein. J. Biol. Chem. 262(5), 2275-2282)). Under physiological conditions, the net reaction is a heteroexchange in which CETP carries triglyceride to HDL from the apoB lipoprotein and transports cholesterol ester from HDL to the apoB lipoprotein.

In humans, CETP plays a role in reverse cholesterol transport, the process whereby cholesterol is returned to the liver from peripheral tissues. Intriguingly, many animals do not possess CETP, including animals that have high HDL levels and are known to be resistant to coronary heart disease, such as rodents (see Guyard-Dangremont, V., et. al., (1998) Phospholipid and cholesteryl ester transfer activities in plasma from 14 vertebrate species. Relation to atherogenesis susceptibility, Comp. Biochem. Physiol. B Biochem. Mol. Biol. 120(3), 517-525). Numerous epidemiologic studies correlating the effects of natural variation in CETP activity with respect to coronary heart disease risk have been performed, including studies on a small number of known human null mutations (see Hirano, K.-I., Yamashita, S. and Matsuzawa, Y. (2000) Pros and cons of inhibiting cholesteryl ester transfer protein, Curr. Opin. Lipidol. 11(6), 589-596). These studies have clearly demonstrated an inverse correlation between plasma HDL-C concentration and CETP activity (see Inazu, A., et. al. (2000) Cholesteryl ester transfer protein and atherosclerosis, Curr. Opin. Lipidol. 11(4), 389-396), leading to the hypothesis that pharmacologic inhibition of CETP lipid transfer activity may be beneficial to humans by increasing levels of HDL-C while lowering LDL-C.

Despite the significant therapeutic advance that statins such as simvastatin and atorvastatin represent, statins only achieve a risk reduction of approximately one-third in the treatment and prevention of atherosclerosis and ensuing atherosclerotic disease events. Currently, few pharmacologic therapies are available that favorably raise circulating levels of HDL-C. Certain statins and some fibrates offer modest HDL-C gains. Niacin provides an effective therapy for raising HDL-C but suffers from patient compliance issues, due in part to side effects such as flushing. Drugs that inhibit CETP (CETP inhibitors) have been under development with the expectation that they will effectively raise HDL cholesterol levels and also reduce the incidence of atherosclerosis in patients. Torcetrapib was the first drug that was tested in a long-term outcomes clinical trial. The clinical trial of torcetrapib was terminated early due to a higher incidence of mortality in patients to whom torcetrapib and atorvastatin were administered concomitantly compared with patients who were treated with atorvastatin alone. The cause of the increased mortality is not completely understood, but it is not believed to be associated with the CETP inhibiting effects of the drug. Dalcetrapib was recently tested in a Phase III outcomes trial, which was terminated early because the interim data did not show a clinical benefit. There were no safety issues detected for dalcetrapib.

Anacetrapib and evacetrapib are CETP inhibitors that are currently being tested in large scale Phase III clinical outcomes trials. Data from the recently completed DEFINE Phase II/III trial of anacetrapib are promising. Patients who were treated with anacetrapib along with baseline statin therapy showed an increase of HDL-C of 138% and a decrease of LDL-C of 40% compared with patients who were treated with just a statin. See: N. Engl. J. Med. 2010: 363: 2406-15*.* The DEFINE study was not carried out on a large enough scale to serve as a pivotal outcomes trial, but the data in the DEFINE trial were sufficient to indicate that an increase in mortality for patients treated with anacetrapib is unlikely.

Additional compounds are still being sought that may have properties that are advantageous compared with the CETP inhibitors that have so far been studied or are currently being studied. Such properties may include, for example, higher potency, reduced off-target activity, better pharmacodynamics, higher bioavailability, or a reduced food effect compared with many of the highly lipophilic compounds that have so far been studied. "Food effect" refers to the variability in exposure to the active drug that occurs depending on when the patient had last eaten, whether or not the drug is administered with food, and the fat content of the food.

### SUMMARY OF THE INVENTION

The subject matter for which protection is sought is as defined in the claims.

The present invention provides novel compounds of Formula Ia as described below and pharmaceutically acceptable salts thereof as well as pharmaceutical compositions containing them. The compounds are for therapy of patient having diseases and conditions that are treated by inhibition of CETP.

In general, references to the compounds of Formula Ia are also meant to include subsets of compounds of Formula Ia as may be described herein, and are also meant to include the specific numbered examples herein.

### DETAILED DESCRIPTION OF THE INVENTION

### Definitions

The terms used herein have their ordinary meaning and the meaning of such terms is independent at each occurrence thereof. That notwithstanding and except where stated otherwise, the following definitions apply throughout the specification and claims. Chemical names, common names, and chemical structures may be used interchangeably to describe the same structure. These definitions apply regardless of whether a term is used by itself or in combination with other terms, unless otherwise indicated. Hence, the definition of "alkyl" applies to "alkyl" as well as the "alkyl" portions of "hydroxyalkyl," "haloalkyl," "-O-alkyl," etc.

As used herein, and throughout this disclosure, the following terms, unless otherwise indicated, shall be understood to have the following meanings:
The term "alkyl," as used herein, refers to an aliphatic hydrocarbon group having one of its hydrogen atoms replaced with a bond having the specified number of carbon atoms. In different embodiments, an alkyl group contains from 1 to 6 carbon atoms (C₁-C₆alkyl) or from 1 to 3 carbon atoms (C₁-C₃alkyl). Non-limiting examples of alkyl groups include methyl, ethyl, *n*-propyl, isopropyl, *n*-butyl, sec-butyl, isobutyl, tert-butyl, *n*-pentyl, neopentyl, isopentyl, n-hexyl, isohexyl, and neohexyl. In one embodiment, an alkyl group is linear. In another embodiment, an alkyl group is branched.
The term "haloalkyl," as used herein, refers to an alkyl group as defined above, wherein one or more of the alkyl group's hydrogen atoms has been replaced with a halo. In one embodiment, a haloalkyl group has from 1 to 6 carbon atoms (C₁-C₆haloalkyl). In another embodiment, a halooalkyl group has from 1 to 3 carbon atoms (C₁-C₃fluoroalkyl). In another embodiment, a haloalkyl group is substituted with from 1 to 3 halogen atoms. Non-limiting examples of ahlooalkyl groups include -CH₂F, -CHF₂, and -CF₃. The term "C₁-C₃ haloalkyl" refers to a halooalkyl group having from 1 to 3 carbon atoms.
The term "alkenyl," as used herein, refers to an aliphatic hydrocarbon group containing at least one carbon-carbon double bond and having one of its hydrogen atoms replaced with a bond. An alkenyl group may be straight or branched and contain from about 2 to about 15 carbon atoms. In one embodiment, an alkenyl group contains from about 2 to 4 carbon atoms. Non-limiting examples of alkenyl groups include ethenyl, propenyl, n-butenyl, 3-methylbut-2-enyl, n-pentenyl, octenyl, and decenyl. The term "C₂-C₆alkenyl" refers to an alkenyl group having from 2 to 6 carbon atoms. The term "C₂-C₄alkenyl" refers to an alkenyl group having from 2 to 4 carbon atoms. Unless otherwise indicated, an alkenyl group is unsubstituted.
The term "alkylene," as used herein, refers to an alkyl group, as defined above, wherein one of the alkyl group's hydrogen atoms has been replaced with a bond. Non-limiting examples of alkylene groups include -CH₂-, -CH₂CH₂-, -CH₂CH₂CH₂-, -CH₂CH₂CH₂CH₂-, -CH(CH₃)CH₂CH₂-, -CH(CH₃)-, and -CH₂CH(CH₃)CH₂-. In one embodiment, an alkylene group has from 1 to about 6 carbon atoms (C₁-C₆alkylene). In another embodiment, an alkylene group has from 1 to 3 carbon atoms (C₁-C₃alkylene). In another embodiment, an alkylene group is branched. In another embodiment, an alkylene group is linear. In one embodiment, an alkylene group is -CH₂-. Unless otherwise indicated, an alkylene group is unsubstituted.
The term "alkynyl," as used herein, refers to an aliphatic hydrocarbon group containing at least one carbon-carbon triple bond, and which may be linear or branched or combinations thereof. Examples of alkynyl include ethynyl, propargyl, 3-methyl-1-pentynyl, 2-heptynyl and the like. Unless otherwise indicated, an alkynyl group is unsubstituted.
The term "alkoxy," as used herein, refers to an -O-alkyl group, wherein an alkyl group is as defined above. Non-limiting examples of alkoxy groups include methoxy, ethoxy, n-propoxy, isopropoxy, *n*-butoxy, and tert-butoxy. An alkoxy group is bonded via its oxygen atom to the rest of the molecule.
The term "aryl," as used herein, refers to an aromatic monocyclic or multicyclic ring system comprising from about 6 to about 14 carbon atoms. In one embodiment, an aryl group contains from about 6 to 10 carbon atoms (C₆-C₁₀aryl). In another embodiment an aryl group is phenyl. Non-limiting examples of aryl groups include phenyl and naphthyl.
The term "composition," as in pharmaceutical composition, is intended to encompass a product comprising the active ingredient(s) and the inert ingredient(s) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of Formula Ia and a pharmaceutically acceptable carrier.
The term "cycloalkyl," as used herein, refers to a saturated ring containing the specified number of ring carbon atoms, and no heteroatom. In a like manner the term "C₃-C₆cycloalkyl" refers to a saturated ring having from 3 to 6 ring carbon atoms. Non-limiting examples of monocyclic cycloalkyls include cyclopropyl, cyclobutyl, cyclopentyl, and cyclohexyl. In some embodiments, this disclosure refers to a cycloalkyl optionally having a specified number of double bonds, *e.g.,* 1-2 double bonds. By way of example, in the case of a cyclohexyl group optionally having 1-2 double bonds, this designation includes a cyclohexyl group, a cyclohexenyl group, and a cyclohexadiene group.
The term "halo" or "halogen," as used herein, means -F, -Cl, -Br or -I. In one embodiment, a halo group is -F or -Cl. In another embodiment, a halo group is -F.
The term "heterocyclyl" or "heterocyclic ring"," as used herein, refers to a fully or partially saturated cyclic compound containing one or more heteroatom groups which may be one or more of N, S, O, S(O), S(O)₂, or (N)R, where R is H or a substituent group, and may have one or more double bonds and one or more carbonyl groups. In general, when heterocycles are defined herein, the definition will include the number of ring members, the number of double bonds and carbonyl groups (if any), and the specific heteroatom groups. The heterocycles in some cases will be aromatic, depending on the number of double bonds (e.g., 6-membered ring with 3 double bonds). Aromatic heterocycles are also referred to as heteroaromatics or heteroaryls. S(O), S(O)₂, and N(R) are referred to as heteroatom groups, and each heteroatom group is counted as one ring member, as is also the case for N, S, and O.
The term "substituted" means that one or more hydrogens on the designated atom/atoms is/are replaced with a selection from the indicated group, provided that the designated atom's normal valency under the existing circumstances is not exceeded, and that the substitution results in a stable compound. Combinations of substituents and/or variables are permissible only if such combinations result in stable compounds. By "stable compound" or "stable structure" is meant a compound that is sufficiently robust to survive isolation from a reaction mixture to a useful degree of purity, and formulation into an efficacious therapeutic agent.
The term "optionally substituted" means that a compound may or may not be substituted with the specified groups, radicals or moieties. Likewise the term "optionally having" means means that a compound may or may not contain the specified groups, radicals, moieties, or double bonds.
A "subject" or "patient" as used herein refers to a human or non-human mammal. In one embodiment, a subject or patient is a human. In another embodiment, the subject or patient is a chimpanzee, dog, or cat.
The term "therapeutically effective amount" as used herein refers to an amount of the compound of Formula I and/or an additional therapeutic agent, or a composition thereof that is effective in producing the desired therapeutic, ameliorative, inhibitory, or preventative effect when administered to a patient suffering from a disease or condition mediated by the inhibition of CETP. In the combination therapies of the present invention, a therapeutically effective amount can refer to each individual agent or to the combination as a whole, wherein the amounts of all agents administered are together effective, but wherein the component agent of the combination may not be present individually in an effective amount.

When any substituent or variable occurs more than one time in any constituent or the compound of Formula I, its definition on each occurrence is independent of its definition at every other occurrence, unless otherwise indicated. For example, description of radicals which include the expression "-N(C₁-₃alkyl)₂" means -N(CH₃)(CH₂CH₃), -N(CH₃)(CH₂CH₂CH₃), and-N(CH₂CH₃)(CH₂CH₂CH₃), as well as -N(CH₃)₂, -N(CH₂CH₃)₂, and -N(CH₂CH₂CH₃)₂.

One or more compounds of the invention may exist in unsolvated as well as solvated forms with pharmaceutically acceptable solvents such as water, ethanol, and the like, and it is intended that the invention embrace both solvated and unsolvated forms. "Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of suitable solvates include ethanolates, methanolates, and the like. "Hydrate" is a solvate wherein the solvent molecule is H₂O.

In the above definitions with multifunctional groups, the attachment point is at the last group, unless otherwise specified on the substituent group by a dash. A dash on the substituent group would then represent the point of attachment.

It should be noted that any carbon as well as heteroatom with unsatisfied valences in the text, schemes, examples and tables herein is assumed to have the sufficient number of hydrogen atom(s) to satisfy the valences.

### Optical Isomers - Diastereomers - Geometric Isomers - Tautomers

The compounds disclosed herein generally have at least two asymmetric centers, and can thus occur as pure stereoisomers and as mixtures of stereoisomers, including racemates, racemic mixtures, single enantiomers, mixtures of enantiomers, diastereomeric mixtures and individual diastereomers. Different stereoisomers having the same 2-dimensional chemical structure may have different levels of activity with respect to CETP inhibition, so that some stereoisomers may have higher activity than others. The compounds that are potent inhibitors of CETP may have utility in patients for raising HDL-C, lowering LDL-C, treating dyslipidemia, and for preventing, treating or delaying the onset of conditions that are related to atherosclerosis. Stereoisomers that have little or no activity may have utility as research tools for better understanding CETP inhibition. All stereoisomers of the claimed compounds thus have utility. The compounds of Formula I may also occur as atropisomers (rotamers) due to hindered rotation, which may be observable by NMR spectroscopy, and in some cases may be stable enough with respect to conversion by bond rotation to other atropisomers that they can be isolated and assayed.

It is also possible that the compound of Formula Ia may exist in different tautomeric forms and all such forms are embraced within the scope of the invention.

### Salts

The term "pharmaceutically acceptable salts" refers to salts prepared from pharmaceutically acceptable non-toxic bases or acids including inorganic or organic bases and inorganic or organic acids. When the compound of Formula I is acidic, salts may be derived from inorganic bases including aluminum, ammonium, calcium, copper, ferric, ferrous, lithium, magnesium, manganic salts, manganous, potassium, sodium, zinc, and the like. Particularly preferred are the ammonium, calcium, magnesium, potassium, and sodium salts. Salts in the solid form may exist in more than one crystal structure, and may also be in the form of hydrates. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, triethanolamine, trimethylamine, tripropylamine, tromethamine, and the like.

When the compound of Formula I is basic, salts may be prepared from pharmaceutically acceptable non-toxic acids, including inorganic and organic acids. Such acids include acetic, adipic, ascorbic, benzenesulfonic, benzoic, camphorsulfonic, citric, diethylacetic, ethanesulfonic, formic, fumaric, gluconic, glutamic, hydrobromic, hydrochloric, isethionic, isonicotinic, lactic, maleic, malic, malonic, mandelic, methanesulfonic, mucic, naphthalenedisulfonic, nitric, oxalic, pamoic, pantothenic, phenylpropionic, phosphoric, pimelic, pivalic, propionic, salicylic, succinic, sulfuric, sulfaminic, tartaric, p-toluenesulfonic acid, trifluoroacetic and the like. Particularly preferred are citric, hydrobromic, hydrochloric, maleic, phosphoric, sulfuric, and tartaric acids.

It will be understood that, as used herein, references to the compounds of Formula I and to the examples are meant to also include the pharmaceutically acceptable salts and prodrugs, where such salts and prodrugs are possible.

### Prodrugs

Prodrugs, which are compounds that are converted to the compound of Formula I as they are being administered to a patient or after they have been administered to a patient, are also compounds of Formula I in the sense that they provide the claimed pharmaceutically active drug moiety to the patient.

### Isotopes

In the compounds of Formula I, the atoms may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the compounds of generic Formula Ia. For example, different isotopic forms of hydrogen (H) include protium (1H) and deuterium (2H). Protium is the predominant hydrogen isotope found in nature. Enriching for deuterium may afford certain therapeutic advantages, such as increasing *in vivo* half-life or reducing dosage requirements, or may provide a compound useful as a standard for characterization of biological samples. Isotopically-enriched compounds within generic Formula I can be prepared without undue experimentation by conventional techniques well known to those skilled in the art or by processes analogous to those described in the Schemes and Examples herein using appropriate isotopically-enriched reagents and/or intermediates.

### Compounds of the Invention

In a first aspect, the invention provides a compound of Formula Ia or a pharmaceutically acceptable salt thereof: 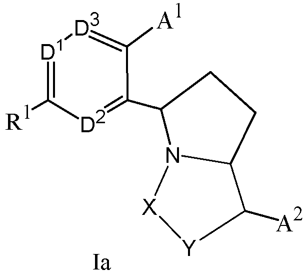
wherein X is -C(=O)-;
Y is CH₂;
R¹ is CF₃;
D¹, D², and D³ are CH;
A¹ is phenyl or pyridyl, wherein A¹ is optionally substituted with 1-3 groups which are each independently F, -OCH₃, or isopropyl, and optionally one substituent group Z;
Z is A³, -CH₂CH₂CO₂R⁸;
R⁸ is H or -CH₃;
A³ is phenyl, cyclohexyl, or pyridyl, wherein A³ is optionally substituted with 1-2 groups -CH₃, and is optionally substituted with 1 group which is -CO₂R⁸; and
A² is phenyl or pyridinyl, wherein A² is substituted with 1 or 2 groups which are each independently CF₃, and F.

In an embodiment, the present invention provides a compound selected from the list below:
2"-((3S,7R,7aS)-7-(3,5-bis(trifluoromethyl)phenyl)-5-oxohexahydro-1H-pyrrolizin-3-yl)-4'-methoxy-2-methyl-4"-(trifluoromethyl)-[1,1':3',1"-terphenyl]-4-carboxylic acid;
4-{5-[2-{(3S,7R,7aS)-7-[3,5-bis(trifluoromethyl)phenyl]-5-oxohexahydro-1H-pyrrolizin-3-yl}-4-(trifluoromethyl)phenyl]-6-methoxypyridin-3-yl}-3,5-dimethylbenzoic acid;
(1R,5S, 7aS)-1-[3,5-bis(trifluoromethyl)phenyl]-5-[4'-fluoro-2'-methoxy-5'-(1-methylethyl)-4-(trifluoromethyl)biphenyl-2-yl]hexahydro-3H-pyrrolizin-3-one;
4-{5-[2-{(3S,7R,7aS)-7-[3,5-bis(trifluoromethyl)phenyl]-5-oxohexahydro-1H-pyrrolizin-3-yl}-4-(trifluoromethyl)phenyl]-6-methoxypyridin-3-yl}-3-methylbenzoic acid;
4-{5-[2-{(3S,7R,7aS)-7-[3-fluoro-5-(trifluoromethyl)phenyl]-5-oxohexahydro-1H-pyrrolizin-3-yl}-4-(trifluoromethyl)phenyl]-6-methoxypyridin-3-yl}-3,5-dimethylbenzoic acid;
(1R,5S,7aS)-5-[4'-fluoro-2'-methoxy-5'-(1-methylethyl)-4-(trifluoromethyl)biphenyl-2-yl]-1-[3-fluoro-5-(trifluoromethyl)phenyl]hexahydro-3H-pyrrolizin-3-one;
4-{5-[2-{(3S,7R,7aS)-7-[3-fluoro-5-(trifluoromethyl)phenyl]-5-oxohexahydro-1H-pyrrolizin-3-yl}-4-(trifluoromethyl)phenyl]-6-methoxypyridin-3-yl}-3-methylbenzoic acid;
2"-{(3S,7R,7aS)-7-[3-fluoro-5-(trifluoromethyl)phenyl]-5-oxohexahydro-1H-pyrrolizin-3-yl}-4'-methoxy-2-methyl-4"-(trifluoromethyl)-1,1':3',1"-terphenyl-4-carboxylic acid; and
4-{6-methoxy-5-[2-{(3S,7R,7aS)-5-oxo-7-[2-(trifluoromethyl)pyridin-4-yl]hexahydro-1H-pyrrolizin-3-yl}-4-(trifluoromethyl)phenyl]pyridin-3-yl}-3,5-dimethylbenzoic acid.

In a further aspect, the present invention provides a pharmaceutical composition comprising a therapeutically effective amount of a compound of the invention, or a pharmaceutically acceptable salt thereof, and a pharmaceutically acceptable carrier.

In a further aspect, the invention relates to a compound of the invention, or a pharmaceutically acceptable salt thereof, for use in any one selected from
a) a method of treating atherosclerosis
b) a method of raising HDL-C; or
c) a method of lowering LDL
d) a method of treating dyslipidemia.

In a further aspect, the invention provides a pharmaceutical composition comprising a compound of the invention, or a pharmaceutically acceptable salt thereof, a pharmaceutically acceptable carrier, and an additional active ingredient selected from:
(i) an HMG-CoA reductase inhibitor;
(ii) a bile acid sequestrant;
(iii) niacin and a related compound;
(iv) a PPAR□ agonist;
(v) a cholesterol absorption inhibitor;
(vi) an acyl CoA:cholesterol acyltransferase (ACAT) inhibitor;
(vii) a phenolic anti-oxidant;
(viii) a microsomal triglyceride transfer protein (MTP)/ApoB secretion inhibitor;
(ix) an anti-oxidant vitamin;
(x) a thyromimetic;
(xi) a LDL (low density lipoprotein) receptor inducer;
(xii) a platelet aggregation inhibitor;
(xiii) vitamin B12 (also known as cyanocobalamin);
(xiv) folic acid or a pharmaceutically acceptable salt or ester thereof;
(xv) an FXR or LXR ligand;
(xvi) an agent that enhances ABCA1 gene expression;
(xvii) an ileal bile acid transporter; or
(xviii) a niacin receptor agonist.

In a further aspect, the present invention provides a compound of Formula Ia selected from the examples described in the Example Section below.

### Utilities

The compounds disclosed herein, including pharmaceutically acceptable salts thereof, are potent inhibitors of CETP. The compounds may therefore be useful in treating mammalian patients, preferably human patients, having diseases and conditions that are treated by inhibition of CETP.

One aspect of the present invention provides a compound of the invention for use in a method for treating or reducing the risk of developing a disease or condition that may be treated or prevented by inhibition of CETP by administering a therapeutically effective amount of the compound of Formula Ia to a patient in need of treatment. The patient is a human or mammal, but is most often a human.

Diseases or conditions that may be treated with the compounds of Formula I, or which the patient may have a reduced risk of developing as a result of being treated with the compounds of Formula I, include: atherosclerosis, peripheral vascular disease, dyslipidemia, hyperbetalipoproteinemia, hypoalphalipoproteinemia, hypercholesterolemia, hypertriglyceridemia, familial-hypercholesterolemia, cardiovascular disorders, angina, ischemia, cardiac ischemia, stroke, myocardial infarction, reperfusion injury, angioplastic restenosis, hypertension, vascular complications of diabetes, obesity, endotoxemia, and metabolic syndrome. There are reports in the scientific literature that suggest that inhibition of CETP may have utility in preventing or slowing the development of Alzheimer's disease. The compounds of Formula I may therefore have utility in preventing or delaying the progression of Alzheimer's disease or other neurodegenerative diseases.

The compounds disclosed herein are particularly effective in raising HDL-C and/or increasing the ratio of HDL-C to LDL-C. The compounds may also be effective in reducing LDL-C, and may be effective in treating dyslipidemia. These changes in HDL-C and LDL-C may be beneficial in treating atherosclerosis, reducing or delaying the development of atherosclerosis, reducing the risk of developing atherosclerosis, or preventing atherosclerosis. The compounds disclosed herein may thus be beneficial in treating atherosclerosis, reducing or delaying the development of atherosclerosis, reducing the risk of developing atherosclerosis, or preventing atherosclerosis.

Likely indications for atherosclerosis and dyslipidemia using the compounds described herein are written below, where the drug product is titled "CETP inhibitor:"

Atherosclerosis. In patients at high risk of cardiovascular events because of existing coronary, cerebrovascular, or peripheral vascular disease, CETP inhibitor coadministered with an HMG-CoA reductase inhibitor is indicated to reduce the risk of coronary mortality, myocardial infarction, coronary revascularization procedures, ischemic stroke, and cardiovascular death.

Dyslipidemia. CETP inhibitor co-administered with a statin is indicated to reduce elevated LDL-C, apolipoprotein B (ApoB), lipoprotein a (Lp(a)), non-HDL-C, and total cholesterol; and increase HDL-C and apolipoprotein A-1 (Apo A-1) in patients with mixed or primary dyslipidemia.

### Administration and Dose Ranges

Any suitable route of administration may be employed for providing a mammal, especially a human, with an effective dose of the compounds described herein. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably the compound of Formula I is administered orally.

When treating the diseases for which the compound of Formula I is indicated, generally satisfactory results are expected when the compound of Formula I is administered at a daily dosage of from about 0.1 milligram to about 1000 milligram in one dose daily or divided into more than one dose per day.

Oral administration will usually be carried out using tablets. Examples of doses in tablets include 0.1 mg, 0.5 mg, 1 mg, 2 mg, 5 mg, 10 mg, 25 mg, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 350 mg, 400 mg, 450 mg, 500 mg, and 1000 mg. Other oral forms can also have the same dosages (e.g. capsules). A preferred dose is likely in the range of 50-200 mg.

### Pharmaceutical Compositions

Another aspect of the present invention provides pharmaceutical compositions which comprise the compound of Formula Ia and a pharmaceutically acceptable carrier. The pharmaceutical compositions of the present invention comprise the compound of Formula Ia or a pharmaceutically acceptable salt as an active ingredient, as well as a pharmaceutically acceptable carrier and optionally other therapeutic ingredients. A pharmaceutical composition may also comprise a prodrug, or a pharmaceutically acceptable salt thereof, if a prodrug is administered. A pharmaceutical composition may also consist essentially of the compound of Formula I, or a pharmaceutically acceptable salt of the compound, and a pharmaceutically acceptable carrier, without other thereapeutic ingredients.

Pharmaceutical compositions may be formulated to be suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, the compound of Formula I can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compound can also be administered intranasally as, for example, liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

The compound of Formula I may also be administered parenterally. Solutions or suspensions of the compound can be prepared in water suitably mixed with a surfactant such as hydroxypropylcellulose. Dispersions can also be prepared in glycerol, liquid polyethylene glycols and mixtures thereof in oils. Under ordinary conditions of storage and use, these preparations may contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g. glycerol, propylene glycol and liquid polyethylene glycol), suitable mixtures thereof, and vegetable oils.

### Combination Therapy

The compound of Formula I, including pharmaceutically acceptable salts thereof, may be used in pharmaceutical combinations with other drugs that may also be useful in the treatment or amelioration of the diseases or conditions for which the compound of Formula I is useful. Such other drugs may be administered, by a route and in an amount commonly used therefor, contemporaneously or sequentially with the compound of Formula I. When the compound of Formula I is used contemporaneously with one or more other drugs, a pharmaceutical composition in unit dosage form containing such other drugs and the compound of Formula I is preferred. However, the combination therapy also includes therapies in which the compound of Formula I and one or more other drugs are administered concomitantly, on the same or different schedules.

When oral formulations are used, the drugs may be combined into a single combination tablet or other oral dosage form, or the drugs may be packaged together as separate tablets or other oral dosage forms. It is also contemplated that when used in combination with one or more other active ingredients, the compound of Formula I and the other active ingredients may be used in lower doses than when each is used singly. Accordingly, the pharmaceutical compositions of the compound of Formula I include those that contain one or more other active ingredients, in addition to the compound of Formula I.

The compound of Formula I will likely be approved initially for coadministration with a statin, which could be administered in the form of a fixed dose combination of the compound of Formula I and a statin. Additional drugs may also be administered in combination with the compound of Formula I and the statin, either by coadministration or in a fixed dose combination. The compound of Formula I and the drugs that are administered with it may be administered as pharmaceutically acceptable salts, as prodrugs, or otherwise formulated for immediate release, extended release, or controlled release, as necessary.

Examples of statins that may be administered in combination with the compound of Formula I include, but are not limited to, (i) simvastatin and lovastatin which are marketed as ZOCOR^{®} and MEVACOR^{®} in lactone prodrug form and function as inhibitors after administration, and (ii) dihydroxy open ring acid HMG-CoA reductase inhibitors such as atorvastatin (particularly the calcium salt sold in LIPITOR^{®}), rosuvastatin (particularly the calcium salt sold in CRESTOR^{®}), pravastatin (particularly the sodium salt sold in PRAVACHOL^{®}), fluvastatin (particularly the sodium salt sold in LESCOL^{®}), and pitavastatin (particularly the calcium salt sold in LIVALO^{®}), and (iii) other statins that may yet be developed. Preferred statins for combination therapy include atorvastatin, rosuvastatin, and simvasatin, or salt forms thereof, as described above.

Cholesterol absorption inhibitors, and particularly ezetimibe (ZETIA^{®}), as well as other cholesterol asorption inhibitors, such as stanol esters, beta-sitosterol, sterol glycosides such as tiqueside, and other azetidinones, may be administered with the compound of Formula I, generally with a statin, as described above. The preferred cholesterol absorbtion inhibitor is ezetimibe. Combinations of the compound of Formula I with a statin and a cholesterol inhibitor, such as ezetimibe, are also contemplated. Preferred 3-component combinations include combinations of the compound of Formula I with simvastatin, atorvastatin, or rosuvastatin in combination with ezetimibe, where the statins may be salt forms or prodrugs as described above. The combination of simvastatin with ezetimibe is currently marketed as VYTORIN^{®}.

Other cholesterol reducing drugs that may be coadministered with the compound of Formula I in addition to HMG-CoA reductase inhibitors (statins) and cholesterol absorption inhibitors include (i) bile acid sequestrants, as for example cholestyramine, colestipol, dialkylaminoalkyl derivatives of a cross-linked dextran, Colestid^{®}, and LoCholest^{®}, (ii) niacin and related compounds, such as nicotinyl alcohol, nicotinamide, and nicotinic acid or a salt thereof, in an immediate release or extended release form, which may optionally be in the form of a combination with a DP-1 antagonist, such as laropiprant, (iii) PPARα agonists, such as gemfibrozil and fenofibric acid derivatives (fibrates), including clofibrate, fenofibrate, bezafibrate, ciprofibrate, and etofibrate, (iv) acyl CoA:cholesterol acyltransferase (ACAT) inhibitors, such as avasimibe and melinamide, and including selective ACAT-1 and ACAT-2 inhibitors and dual inhibitors, (v) phenolic anti-oxidants, such as probucol, (vi) microsomal triglyceride transfer protein (MTP)/ApoB secretion inhibitors, (vii) anti-oxidant vitamins, such as vitamins C and E and beta carotene, (viii) thyromimetics, (ix) LDL (low density lipoprotein) receptor inducers, (x) platelet aggregation inhibitors, for example glycoprotein IIb/IIIa fibrinogen receptor antagonists and aspirin, (xi) vitamin B12 (also known as cyanocobalamin), (xii) folic acid or a pharmaceutically acceptable salt or ester thereof, such as the sodium salt and the methylglucamine salt, (xiii) FXR and LXR ligands, including both inhibitors and agonists, (xiv) agents that enhance ABCA1 gene expression, (xv) ileal bile acid transporters, and (xvi) niacin receptor agonists (e.g. acipimox and acifran) and partial agonists.

Finally the compound of Formula I can be combined with compounds that are useful for treating other diseases, such as diabetes, hypertension and obesity, as well as other anti-atherosclerotic compounds. Such combinations may be used to treat one or more of such diseases as diabetes, obesity, atherosclerosis, and dyslipidemia, or more than one of the diseases associated with metabolic syndrome. The combinations may exhibit synergistic activity in treating these diseases, allowing for the possibility of administering reduced doses of active ingredients, such as doses that otherwise might be sub-therapeutic.

Examples of other active ingredients that may be administered in combination with a compound of Formula I include, but are not limited to, compounds that are primarily antidiabetic compounds, including:
(a) PPAR gamma agonists and partial agonists, including glitazones and nonglitazones (e.g. pioglitazone, englitazone, MCC-555, rosiglitazone, balaglitazone, netoglitazone, T-131, LY-300512, LY-818, and compounds described in WO 02/060388, WO 02/08188, WO 2004/019869, WO 2004/020409, WO 2004/020408, and WO2004/066963);
(b) biguanides such as metformin, phenformin, and pharmaceutically acceptable salts thereof, in particular metformin hydrochloride and extended release formulations thereof, such as Glumetza^{™}, Fortamet^{™}, and GlucophageXR^{™};
(c) protein tyrosine phosphatase-IB (PTP-1B) inhibitors, such as ISIS-113715 and TTP814;
(d) dipeptidyl peptidase IV (DP-IV) inhibitors, including sitagliptin, vildagliptin, saxagliptin, alogliptin, linagliptin, dutogliptin, teneligliptin, omarigliptin, and gemigliptin;
(e) insulin or insulin mimetics, such as for example insulin lispro, insulin glargine, insulin detemir, insulin glulisine, insulin degludec, SBS1000, insulin zinc suspension, and oral and inhalable formulations of insulin and insulin analogs;
(f) sulfonylureas, such as tolbutamide, glipizide, glimepiride, acetohexamide, chlorpropamide, glibenclamide, and related materials;
(g) α-glucosidase inhibitors (such as acarbose, adiposine; camiglibose; emiglitate; miglitol; voglibose; pradimicin-Q; and salbostatin);
(h) PPARα/γ dual agonists, such as muraglitazar, tesaglitazar, farglitazar, and naveglitazar;
(i) PPARδ agonists such as GW501516 and those disclosed in WO97/28149;
(j) glucagon receptor antagonists;
(k) GLP-1, GLP-1 derivatives, GLP-1 mimetics, GLP-1 analogs, and GLP-1 receptor agonists, such as exendins, e.g. exenatide (BYETTA), dulaglutide, semaglutide, albiglutide, liraglutide, lixisenatide, and taspoglutide, including intranasal, transdermal, and once weekly formulations thereof, and oxyntomodulin analogs and derivatives, and non-peptidyl GLP-1 receptor agonists;
(l) GIP-1;
(m) amylin and amylin analogs (e.g. pramlintide);
(n) Non-sulfonylurea insulin secretagogues, such as the meglitinides (e.g. glimepiride, mitiglinide, meglitinide, nateglinide, and rapeglinide);
(o) leptin and leptin derivatives and agonists; and
(p) SGLT2 inhibitors, including canagliflozin, dapagliflozin, ipragliflozin, empagliflozin, tofogliflozin, luseogliflozin (TS-071), ertugliflozin, and remogliflozin,

Preferred combinations with antidiabetic compounds include combinations of the compounds disclosed herein with DP-IV inhibitors (sitagliptin, vildagliptin, saxagliptin, alogliptin, linagliptin, dutogliptin, teneligliptin, omarigliptin, and gemigliptin), combinations with biguanides, and combinations with both a DP-IV inhibitor and a biguanide. The preferred DP-IV inhibitor is sitagliptin, and the preferred biguanide is metformin in the formulations and salt forms described above.

Other active ingredients that may be used in combination with the compound of Formula I include antiobesity compounds, including 5-HT(serotonin) inhibitors, neuropeptide Y5 (NPY5) inhibitors, melanocortin 4 receptor (Mc4r) agonists, cannabinoid receptor 1 (CB-1) antagonists/inverse agonists, and β₃ adrenergic receptor agonists. These are listed in more detail later in this section.

These other active ingredients also include active ingredients that are used to treat inflammatory conditions, such as aspirin, non-steroidal anti-inflammatory drugs, glucocorticoids, azulfidine, and selective cyclooxygenase-2 (COX-2) inhibitors, including etoricoxib, celecoxib, rofecoxib, and Bextra.

Antihypertensive compounds may also be used advantageously in combination therapy with the compound of Formula I. Examples of antihypertensive compounds that may be used with the compound of Formula I include thiazide-like diuretics, e.g., hydrochlorothiazide (HCTZ or HCT); angiotensin converting enzyme inhibitors (e.g, alacepril, benazepril, captopril, ceronapril, cilazapril, delapril, enalapril, enalaprilat, fosinopril, imidapril, lisinopril, moveltipril, perindopril, quinapril, ramipril, spirapril, temocapril, or trandolapril); dual inhibitors of angiotensin converting enzyme (ACE) and neutral endopeptidase (NEP) such as omapatrilat, sampatrilat and fasidotril; angiotensin II receptor antagonists, also known as angiotensin receptor blockers or ARBs, which may be in free-base, free-acid, salt or pro-drug form, such as azilsartan, e.g., azilsartan medoxomil potassium (EDARBI^{®}), candesartan, e.g., candesartan cilexetil (ATACAND^{®}), eprosartan, e.g., eprosartan mesylate (TEVETAN^{®}), irbesartan (AVAPRO^{®}), losartan, e.g., losartan potassium (COZAAR^{®}), olmesartan, e.g, olmesartan medoximil (BENICAR^{®}), telmisartan (MICARDIS^{®}), valsartan (DIOVAN^{®}), and any of these drugs used in combination with a thiazide-like diuretic such as hydrochlorothiazide (e.g., HYZAAR^{®}, DIOVAN HCT^{®}, ATACAND HCT^{®}), etc.); potassium sparing diuretics such as amiloride HCl, spironolactone, epleranone, triamterene, each with or without HCTZ; carbonic anhydrase inhibitors, such as acetazolamide; neutral endopeptidase inhibitors (e.g., thiorphan and phosphoramidon); aldosterone antagonists; aldosterone synthase inhibitors; renin inhibitors (e.g. urea derivatives of di- and tri-peptides (See U.S. Pat. No. 5,116,835), amino acids and derivatives (U.S. Patents 5,095,119 and 5,104,869), amino acid chains linked by non-peptidic bonds (U.S. Patent 5,114,937), di- and tri-peptide derivatives (U.S. Patent 5,106,835), peptidyl amino diols (U.S. Patents 5,063,208 and 4,845,079) and peptidyl beta-aminoacyl aminodiol carbamates (U.S. Patent 5,089,471); also, a variety of other peptide analogs as disclosed in the following U.S. Patents 5,071,837; 5,064,965; 5,063,207; 5,036,054; 5,036,053; 5,034,512 and 4,894,437, and small molecule renin inhibitors (including diol sulfonamides and sulfinyls (U.S. Patent 5,098,924), N-morpholino derivatives (U.S. Patent 5,055,466), N-heterocyclic alcohols (U.S. Patent 4,885,292) and pyrolimidazolones (U.S. Patent 5,075,451); also, pepstatin derivatives (U.S. Patent 4,980,283) and fluoro- and chloro-derivatives of statone-containing peptides (U.S. Patent 5,066,643); enalkrein; RO 42-5892; A 65317; CP 80794; ES 1005; ES 8891; SQ 34017; aliskiren (2(S),4(S),5(S),7(S)-N-(2-carbamoyl-2-methylpropyl)-5-amino-4-hydroxy-2,7-diisopropyl-8-[4-methoxy-3-(3-methoxypropoxy)-phenyl]-octanamid hemifumarate) SPP600, SPP630 and SPP635); endothelin receptor antagonists; vasodilators (e.g. nitroprusside); calcium channel blockers (e.g., amlodipine, nifedipine, verapamil, diltiazem, felodipine, gallopamil, niludipine, nimodipine, nicardipine, bepridil, nisoldipine); potassium channel activators (e.g., nicorandil, pinacidil, cromakalim, minoxidil, aprilkalim, loprazolam); sympatholitics; beta-adrenergic blocking drugs (e.g., acebutolol, atenolol, betaxolol, bisoprolol, carvedilol, metoprolol, metoprolol tartate, nadolol, propranolol, sotalol, timolol); alpha adrenergic blocking drugs (e.g., doxazocin, prazocin or alpha methyldopa); central alpha adrenergic agonists; peripheral vasodilators (e.g. hydralazine); and nitrates or nitric oxide donating compounds, e.g. isosorbide mononitrate.

Preferred antihypertensives that may be used in combination with the CETP inhibitors disclosed herein include one or more of an angiotensin II antagonist (losartan), an ACE inhibitor (enalapril or captopril), and hydrochlorothiazide.

Anti-obesity compounds may be administered in combination with the compounds of Formula I, including: (1) growth hormone secretagogues and growth hormone secretagogue receptor agonists/antagonists, such as NN703 and hexarelin; (2) protein tyrosine phosphatase-lB (PTP-1B) inhibitors; (3) cannabinoid receptor ligands, such as cannabinoid CB₁ receptor antagonists or inverse agonists, such as rimonabant (Sanofi Synthelabo), AMT-251, and SR-14778 and SR 141716A (Sanofi Synthelabo), SLV-319 (Solvay), BAY 65-2520 (Bayer); (4) anti-obesity serotonergic agents, such as fenfluramine, dexfenfluramine, phentermine, and sibutramine; (5) β3-adrenoreceptor agonists, such as AD9677/TAK677 (Dainippon/Takeda), CL-316,243, SB 418790, BRL-37344, L-796568, BMS-196085, BRL-35135A, CGP12177A, BTA-243, Trecadrine, Zeneca D7114, and SR 59119A; (6) pancreatic lipase inhibitors, such as orlistat (Xenical^{®}), Triton WR1339, RHC80267, lipstatin, tetrahydrolipstatin, teasaponin, and diethylumbelliferyl phosphate; (7) neuropeptide Y1 antagonists, such as BIBP3226, J-115814, BIBO 3304, LY-357897, CP-671906, and GI-264879A; (8) neuropeptide Y5 antagonists, such as GW-569180A, GW-594884A, GW-587081X, GW-548118X, FR226928, FR 240662, FR252384, 1229U91, GI-264879A, CGP71683A, LY-377897, PD-160170, SR-120562A, SR-120819A and JCF-104; (9) melanin-concentrating hormone (MCH) receptor antagonists; (10) melanin-concentrating hormone 1 receptor (MCH1R) antagonists, such as T-226296 (Takeda); (11) melanin-concentrating hormone 2 receptor (MCH2R) agonist/antagonists; (12) orexin-1 receptor antagonists, such as SB-334867-A; (13) melanocortin agonists, such as Melanotan II; (14) other Mc4r (melanocortin 4 receptor) agonists, such as CHIR86036 (Chiron), ME-10142, and ME-10145 (Melacure), CHIR86036 (Chiron); PT-141, and PT-14 (Palatin); (15) 5HT-2 agonists; (16) 5HT2C (serotonin receptor 2C) agonists, such as BVT933, DPCA37215, WAY161503, and R-1065; (17) galanin antagonists; (18) CCK agonists; (19) CCK-A (cholecystokinin -A) agonists, such as AR-R 15849, GI 181771, JMV-180, A-71378, A-71623 and SR146131; (20) GLP-1 agonists; (21) corticotropin-releasing hormone agonists; (22) histamine receptor-3 (H3) modulators; (23) histamine receptor-3 (H3) antagonists/inverse agonists, such as hioperamide, 3-(1H-imidazol-4-yl)propyl N-(4-pentenyl)carbamate, clobenpropit, iodophenpropit, imoproxifan, and GT2394 (Gliatech); (24) β-hydroxy steroid dehydrogenase-1 inhibitors (11β-HSD-1 inhibitors), such as BVT 3498 and, BVT 2733, (25) PDE (phosphodiesterase) inhibitors, such as theophylline, pentoxifylline, zaprinast, sildenafil, amrinone, milrinone, cilostamide, rolipram, and cilomilast; (26) phosphodiesterase-3B (PDE3B) inhibitors; (27) NE (norepinephrine) transport inhibitors, such as GW 320659, despiramine, talsupram, and nomifensine; (28) ghrelin receptor antagonists; (29) leptin, including recombinant human leptin (PEG-OB, Hoffman La Roche) and recombinant methionyl human leptin (Amgen); (30) leptin derivatives; (31) BRS3 (bombesin receptor subtype 3) agonists such as [D-Phe6,beta-Ala11,Phe13,Nle14]Bn(6-14) and [D-Phe6,Phe13]Bn(6-13)propylamide; (32) CNTF (Ciliary neurotrophic factors), such as GI-181771 (Glaxo-SmithKline), SR146131 (Sanofi Synthelabo), butabindide, PD170,292, and PD 149164 (Pfizer); (33) CNTF derivatives, such as axokine (Regeneron); (34) monoamine reuptake inhibitors, such as sibutramine; (35) UCP-1 (uncoupling protein-1, 2, or 3) activators, such as phytanic acid, 4-[(E)-2-(5,6,7,8-tetrahydro-5,5,8,8-tetramethyl-2-napthalenyl)-1-propenyl]benzoic acid (TTNPB), and retinoic acid; (36) thyroid hormone β agonists, such as KB-2611 (KaroBioBMS); (37) FAS (fatty acid synthase) inhibitors, such as Cerulenin and C75; (38) DGAT1 (diacylglycerol acyltransferase 1) inhibitors; (39) DGAT2 (diacylglycerol acyltransferase 2) inhibitors; (40) ACC2 (acetyl-CoA carboxylase-2) inhibitors; (41) glucocorticoid antagonists; (42) acyl-estrogens, such as oleoyl-estrone; (43) dicarboxylate transporter inhibitors; (44) peptide YY, PYY 3-36, peptide YY analogs, derivatives, and fragments such as BIM-43073D, BIM-43004C, (45) Neuropeptide Y2 (NPY2) receptor agonists such NPY3-36, N acetyl [Leu(28,31)] NPY 24-36, TASP-V, and cyclo-(28/32)-Ac-[Lys28-Glu32]-(25-36)-pNPY; (46) Neuropeptide Y4 (NPY4) agonists such as pancreatic peptide (PP); (47) Neuropeptide Y1 (NPY1) antagonists such as BIBP3226, J-115814, BIBO 3304, LY-357897, CP-671906, and GI-264879A; (48) Opioid antagonists, such as nalmefene (Revex ^{®}), 3-methoxynaltrexone, naloxone, and naltrexone; (49) glucose transporter inhibitors; (50) phosphate transporter inhibitors; (51) 5-HT (serotonin) inhibitors; (52) beta-blockers; (53) Neurokinin-1 receptor antagonists (NK-1 antagonists); (54) clobenzorex; (55) cloforex; (56) clominorex; (57) clortermine; (58) cyclexedrine; (59) dextroamphetamine; (60) diphemethoxidine, (61) N-ethylamphetamine; (62) fenbutrazate; (63) fenisorex; (64) fenproporex; (65) fludorex; (66) fluminorex; (67) furfurylmethylamphetamine; (68) levamfetamine; (69) levophacetoperane; (70) mefenorex; (71) metamfepramone; (72) methamphetamine; (73) norpseudoephedrine; (74) pentorex; (75) phendimetrazine; (76) phenmetrazine; (77) picilorex; (78) phytopharm 57; (79) zonisamide, (80) aminorex; (81) amphechloral; (82) amphetamine; (83) benzphetamine; and (84) chlorphentermine.

The combination therapies described above which use the compounds of Formula I may also be useful in the treatment of the metabolic syndrome. According to one widely used definition, a patient having metabolic syndrome is characterized as having three or more symptoms selected from the following group of five symptoms: (1) abdominal obesity; (2) hypertriglyceridemia; (3) low high-density lipoprotein cholesterol (HDL); (4) high blood pressure; and (5) elevated fasting glucose, which may be in the range characteristic of Type 2 diabetes if the patient is also diabetic. Each of these symptoms is defined clinically in the Third Report of the National Cholesterol Education Program Expert Panel on Detection, Evaluation and Treatment of High Blood Cholesterol in Adults (Adult Treatment Panel III, or ATP III), National Institutes of Health, 2001, NIH Publication No. 01-3670. Patients with metabolic syndrome have an increased risk of developing the macrovascular and microvascular complications that are listed above, including atherosclerosis and coronary heart disease. The combinations described above may ameliorate more than one symptom of metabolic syndrome concurrently (e.g. two symptoms, three symptoms, four symptoms, or all five of the symptoms).

### ASSAYS

### Protocol: Scintillation Proximity Assay (SPA) for CETP activity

First, low density lipoprotein (LDL) (Meridian) was biotinylated by incubating LDL with biotin for 1 hour on ice, after which it was dialyzed to remove free biotin. Then compounds at varying concentrations were incubated with 15 nM CETP (reagent production group, In Vitro Pharmacology, MRL Rahway) and 50 ug/ml of the biotinylated LDL in 50 mM HEPES, 150 mM NaCl, pH 7.4, for 1 hour at 37°C. The reaction was started by adding ³H-cholesterol ester high density lipoprotein (HDL) (American Radiochemicals Corp) at a concentration of ~ 0.6 nM. The reaction proceeded for 2 hours at 37°C, after which time it was quenched by the addition of 12% acetic acid. PVT streptavadin-coated scintillation proximity beads, which had been brought to room temperature, were then added at a concentration of 4 mg/ml. The assay was then mixed and counted after one half hour in a Microbeta plate reader.

### In vitro radioactive assays of CETP-catalyzed CE and TG transfer (RTA assay)

Reagents and sources are: [3H] cholesteryl oleate (GE #TRK.886), [3H] Triolein (Perkin-Elmer NET-431), Butylated hydroxyl toluene (Aldrich, #D4740-4), DOPC (Sigma, # P6354), Sodium Bromide (Fisher scientific #S255-500), PEG 8000 (Fisher, #BP233-1), and human HDL (Intracel Corp #RP-036).

An in vitro assay for determining IC₅₀'s to identify compounds that inhibit CETP transfer activity is performed based on a modification of a published method (Morton and Zilversmit, (1981) A plasma inhibitor of triglyceride and cholesteryl ester transfer activities, J. Biol. Chem. 256(23), 11992-11995). The ability of inhibitors to alter CETP activity is performed using two different assays: one using recombinant CETP and one using an endogenous plasma source of CETP. Both assays measure the transfer of [3H] cholesteryl oleate or [3H] triolein from exogenous LDL to HDL.

Radiolabeled donor particles are generated by first combining 100 µl of 200 µM butylated hydroxyl toluene in CHCl₃, 216 µL of 21.57 mM DOPC in EtOH, and either 500 µCi [3H]-triolein (Perkin Elmer #NET-431) or 500 µCi [3H]-cholesteryl oleate (GE #TRK886) in a glass tube. Reagents are mixed, dried under nitrogen, and then resuspended in 2 mL of 50 mM Tris, 27 µM EDTA at pH 7.4. After a brief vortex, the solution is sonicated until clear and mixed with 20 mL of fresh human serum. The mixture is incubated overnight at 37°C. The [3H] labeled LDL substrate is separated at 1.063 g/ml density by sequential ultracentrifugal flotation in NaBr according to the method of Havel, Eder, et al., 1955, and Chapman, Goldstein, et al., 1981. Once isolated the particles are dialyzed 3x in CETP buffer (50 mM Tris, pH 7.4, 100 mM NaCl, 1 mM EDTA). Human HDL is purchased from Intracel and used as the acceptor particles.

Transfer assays are performed in a 96-well v-bottom polypropylene plate. For the RTA using recombinant CETP (2% RTA), an assay cocktail is prepared with the final concentrations 128 µg/mL HDL, 20 nM rCETP, 2% human serum, and 1 × CETP buffer. 1 µL of each test compound diluted in DMSO is added to 47 µL of assay cocktail per well and incubated at 37°C for 1 hour. To initiate the transfer reaction, 2 µL radiolabeled LDL is added. After an additional 60 min of incubation at 37°C, the transfer action is terminated by precipitation of LDL with an equal volume of 20% W/V PEG 8000. The plates are centrifuged at 2000 rpm for 30 minutes at 4°C. A 40 µL aliquot of the HDL-containing supernatant is transferred to a Packard Optiplate^{™} with 200 µL of MicroScint^{™} 20. After mixing, plates are counted by liquid scintillation. Counts present in the supernatant for blanks (wells containing only HDL acceptor, CETP buffer and DMSO) are subtracted from those containing test compounds and used to correct for nonspecific transfer.

For the transfer assay using endogenous CETP from serum (95% RTA), the same procedure is used except that human serum is added such that a final concentration of serum of 95% of the total assay volume is achieved, yielding a concentration of approximately 15 nM endogenous CETP in the assay. This is then combined with HDL and CETP buffer and the reaction proceeds as above and is terminated as described.

Comparison of the counts of samples with inhibitors to an uninhibited (DMSO only) positive control yield a percent inhibition. A plot of percent inhibition vs. log of inhibitor concentration, fit to a Sigmoidal 4 parameter equation is used to calculate IC₅₀.

### EXAMPLES

The following schemes and examples are provided so that the invention will be more fully appreciated and understood. These examples are illustrative and are not to be construed as limiting the invention in any way. The claims appended hereto define the scope of the invention. The examples are specific compounds of the invention. The claims describe compounds of the invention in a more general way.

Starting materials are commercially available or are made using known procedures or as shown below. The examples may be synthesized using the general schemes provided below. The data reported for the examples below were obtained using the RTA assay in 95% human serum. The IC₅₀'s for the examples using this assay are in the range of about 20-5000 nM. Preferred compounds have an IC₅₀ less than about 500 nM. More preferred compounds have IC₅₀ values of less than about 200 nM. When compounds of Formula I are mentioned herein, such compounds include compounds defined generically by Formula I and also the specific examples disclosed herein. The specific compounds that are disclosed as examples are CETP inhibitors. They were made as disclosed, and they inhibit CETP as shown by the assay data that were obtained for the individual compounds.

The following solvents, reagents, protecting groups, moieties, and other desginations may be referred to by their abbreviations as follows:
Me = methyl; Et = ethyl, Pr = propyl; iPr = isopropyl; Bu = butyl; t-Bu = tertiary butyl; Ph = phenyl, and Ac = acetyl
"APCI" is atmospheric pressure chemical ionization.
"Boc" is *tert*-butoxycarbonyl.
"n-BuLi" is n-butyl lithium.
"calc'd" is calculated.
"DIPEA" and "DIEA" are N,N-diisopropylethylamine.
"DCM" is dichloromethane.
"DIEA" is diisopropylethylamine.
"DMF" is N,N-dimethylformamide.
"DMA" is dimethylacetamide.
"DMAP" is 4-dimethylaminopyridine.
"DMSO" is dimethyl sulfoxide.
"DOPC" is 1,2-dioleoyl-*sn*-glycero-3-phosphocholine
"EDTA" is ethylenediaminetetraacetic acid.
"EtOAc" is ethyl acetate.
"EtOH" is ethanol.
"h" represents hour or hours.
"HPLC" is high pressure liquid chromatography.
"HS" is human serum.
"IPA" is isopropyl alcohol.
"K-Selectride^{®} is potassium tri-sec-butylborohydride reagent commercially available from Sigma-Aldrich.
"LCMS" is liquid chromatography mass spectrometry.
"LiHMDS" is lithium hexamethyldisilazide.
"MeCN" is acetonitrile.
"MeOH" is methanol.
"min" means minute of minutes.
"NCS" is N-chlorosuccinamide.
"OAc" is acetate.
"Pd₂dba₃" is Tris(dibenzylideneacetone)dipalladium(0), a catalyst precursor.
"PEG" is poly(ethylene glycol).
"RT" and "rt" are abbreviations for room temperature.
"SFC" is supercritical fluid chromatography.
"TEA" is triethylamine.
"TFA" is trifluoroacetic acid.
"Tf₂O" is an abbreviation for trifluoromethanesulfonic anhydride.
"THF" is tetrahydrofuran.
"TLC" is thin layer chromatography
"X-Phos" is 2-dicyclohexylphosphino-2',4',6'-triisopropylbiphenyl.

### Synthetic Schemes

### Syntheses of Intermediates

The examples were synthesized according to the general schemes shown below. Synthetic intermediates for making the compounds are made as described below and are illustrated in the following schemes. The various starting materials used in the schemes are commercially available or are readily made by persons skilled in the art.

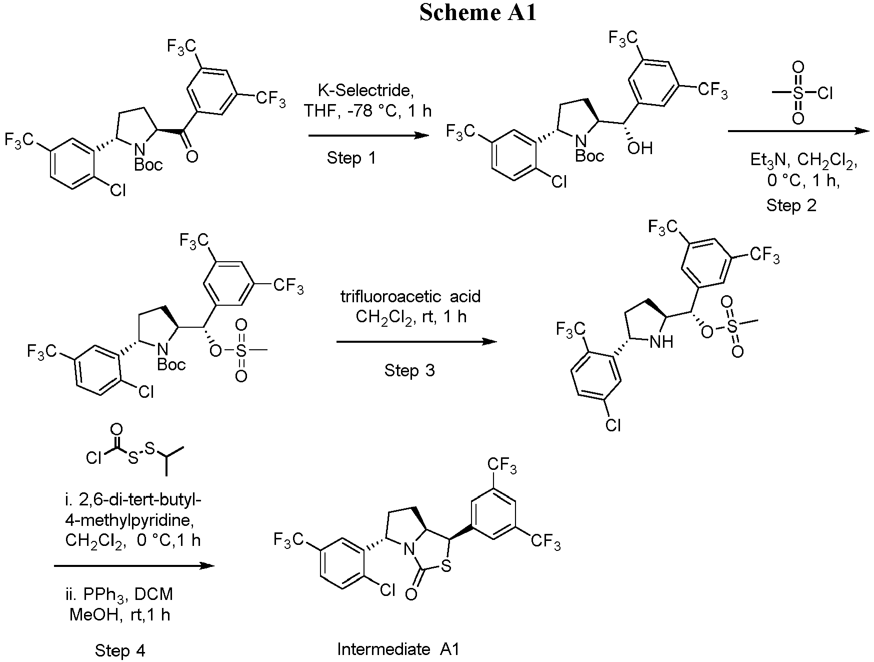

(2S,5S)-tert-butyl2-(3,5-bis(trifluoromethyl)benzoyl)-5-(2-chloro-5-(trifluoromethyl)phenyl)pyrrolidine-1-carboxylate was reduced to a secondary alcohol, which was converted to a leaving group (Mesylate). After Boc deprotection, the pyrrolidine amine converted to carbo(dithioperoxoate) derivative. Upon cleavage of disulfide bond with triphenylphosphine , it cyclizes to give tetrahydropyrrolo[1,2-c]thiazol-3(1H)-one compound (intermediate A1).

### Intermediate A1

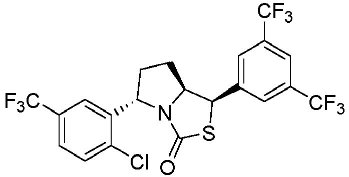

### (2S,5S)-tert-butyl2-((S)-(3,5-bis(trifluoromethyl)phenyl)(hydroxy)methyl)-5-(2-chloro-5-(trifluoromethyl)phenyl)pyrrolidine-1-carboxylate

**Step 1:** A solution of (2S,5S)-tert-butyl 2-(3,5-bis(trifluoromethyl)benzoyl)-5-(2-chloro-5-(trifluoromethyl)phenyl)pyrrolidine-1-carboxylate (500 mg, 0.848 mmol) in THF (30 mL) was cooled to -78 °C. K-Selectride (1.695 mL, 1.695 mmol) was added dropwise. The reaction was stirred at -78 °C for 1 hour. The reaction was diluted with EtOAc, quenched with NH₄Cl solution, washed with brine, dried over Na₂SO₄, filtered and concentrated. The residue was purified by column chromatography to obtain (2S,5S)-tert-butyl 2-((S)-(3,5-bis(trifluoromethyl)phenyl)(hydroxy)methyl)-5-(2-chloro-5-(trifluoromethyl)phenyl)pyrrolidine-1-carboxylate. ¹H NMR (500 MHz, CDCl₃): δ 7.87 (s, 2 H); 7.85 (s, 1 H); 7.45-7.50 (m, 2 H); 7.22 (s, 1 H); 5.44 (d, J = 3.5 Hz, 1 H); 5.24 (d, J = 8.4 Hz, 1 H); 5.06 (dd, J = 8.1, 3.5 Hz, 1 H); 4.54 (t, J = 8.1 Hz, 1 H); 2.04-2.07 (m, 3 H, merged with solvent peak); 1.81-1.85 (m, 1 H); 1.62-1.70 (m, 1H); 1.55-1.59 (m, 3 H, merged with water peak); 1.19-1.20 (m, 9 H).

**Step 2:** To a solution of (2S,5S)-tert-butyl 2-((S)-(3,5-bis(trifluoromethyl)phenyl)(hydroxy)methyl)-5-(2-chloro-5-(trifluoromethyl)phenyl)pyrrolidine-1-carboxylate (100 mg, 0.169 mmol) in DCM (1.5 mL) was added Et₃N (0.031 mL, 0.220 mmol). The resulting solution was kept at 0°C and methanesulfonyl chloride (0.014 mL, 0.186 mmol) was added. The reaction mixture was stirred for 30 minutes. It was diluted with DCM, washed sequentially with NH₄Cl and brine, dried over Na₂SO₄, filtered and concentrated. The residue was purified by column chromatography to obtain (2S,5S)-tert-butyl 2-((S)-(3,5-bis(trifluoromethyl)phenyl)((methylsulfonyl)oxy)methyl)-5-(2-chloro-5-(trifluoromethyl)phenyl)pyrrolidine-1-carboxylate. ¹H NMR (500 MHz, CDCl₃): δ 7.96-7.97 (m, 1 H); 7.82 (s, 2 H); 7.45 (s, 2H); 7.20 (s, 1 H); 6.42 (d, J = 4.9 Hz, 1 H); 4.74-4.79 (m, 2 H); 3.17 (s, 3 H);1.96-2.10 (m, 4H); 1.41-1.45 (m, 1 H); 1.21 (s, 8 H).

**Step 3:** To a solution of (2S,5S)-tert-butyl 2-((S)-(3,5-bis(trifluoromethyl)phenyl)((methylsulfonyl)oxy)methyl)-5-(2-chloro-5-(trifluoromethyl)phenyl)pyrrolidine-1-carboxylate (1.5 g, 2.239 mmol) in DCM (3 mL) was added TFA (2.59 mL, 33.6 mmol). The reaction was stirred for 45 minutes. The volatiles were removed and the residue was dissolved in EtOAc. The resulting mixture was washed with saturated NaHCO₃, then brine, dried over Na₂SO₄, filtered and concentrated to afford (S)-(3,5-bis(trifluoromethyl)phenyl)((2S,5S)-5-(2-chloro-5-(trifluoromethyl)phenyl)pyrrolidin-2-yl)methyl methanesulfonate. It was used for the next step without further purification.

**Step 4:** To a solution of (S)-(3,5-bis(trifluoromethyl)phenyl)((2S,5S)-5-(2-chloro-5-trifluoromethyl)phenyl)pyrrolidin-2-yl)methyl methanesulfonate (170 mg, 0.298 mmol) in DCM (3 mL), was added 2,6-di-tert-butyl-4-methylpyridine (123 mg, 0.597 mmol), followed by SS-isopropyl carbonochlorido(dithioperoxoate) (102 mg, 0.597 mmol) at 0 °C . The reaction was stirred for 30 minutes. To the resulting mixture was added MeOH (1.00 mL) followed by triphenylphosphine (156 mg, 0.597 mmol). The reaction mixture was stirred for 30 minutes. The volatiles were removed. The reaction was diluted with DCM, neutralized with satd. NaHCO₃, washed with brine, dried over Na₂SO₄, filtered and concentrated. The residue was purified by column chromatography to afford (1R,5S,7aS)-1-(3,5-bis(trifluoromethyl)phenyl)-5-(2-chloro-5-(trifluoromethyl)phenyl)tetrahydropyrrolo[1,2-c]thiazol-3(1H)-one. ¹H NMR (500 MHz, CDCl₃): δ 7.95 (s, 2 H); 7.93 (s, 1 H); 7.56 (s, 1 H); 7.52 (s, 2 H); 5.35 (t, J = 7.9 Hz, 1 H); 5.30 (d, J = 8.2 Hz, 1 H); 4.86-4.91 (m, 1 H); 2.75-2.81 (m, 1 H); 1.78-1.86 (m, 1 H); 1.54-1.62 (m, 2 H); 1.33-1.41 (m, 1 H). MS ESI/APCI calc'd. for C21H13ClF9NOS [M + H]+ 533.8, found 533.8.

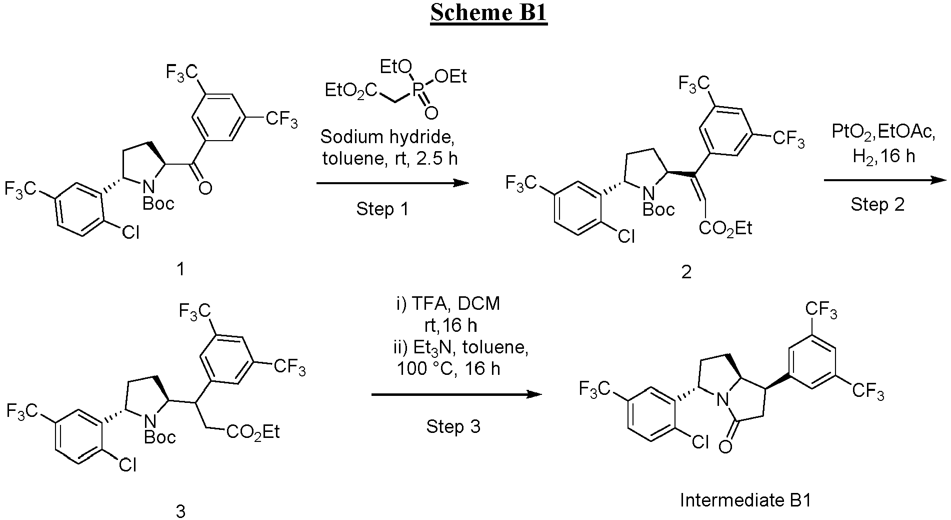

(2S,5S)-tert-butyl2-(3,5-bis(trifluoromethyl)benzoyl)-5-(2-chloro-5-(trifluoromethyl)phenyl)pyrrolidine-1-carboxylate underwent Horner-Wadsworth-Emmons reaction with ethyl 2-(diethoxyphosphoryl)acetate to obtain compound 2, and which was hydrogenated with platinum oxide as the catalyst to give 3. Upon treatment with TFA, and then with Et₃N, compound 3 underwent intramolecular lactam formation to yield (1R,5S,7aS)-1-(3,5-bis(trifluoromethyl)phenyl)-5-(2-chloro-5-(trifluoromethyl)phenyl)hexahydro-3H-pyrrolizin-3-one compound (intermediate B1).

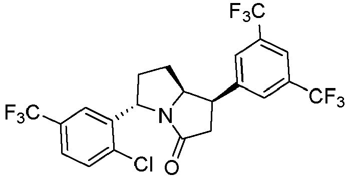

### (1R,5S,7aS)-1-(3,5-bis(trifluoromethyl)phenyl)-5-(2-chloro-5-(trifluoromethyl)phenyl)hexahydro-3H-pyrrolizin-3-one

### Intermediate B1

**Step 1:** To a solution of triethyl phosphonoacetate (3.8 mL, 16.9 mmol) in toluene (40.0 mL) was added sodium hydride (0.68 g, 16.9 mmol) at 0 °C. The resulting solution was stirred at room temperature for 0.5 h. The reaction was cooled back to 0 °C, tert-butyl (2S, 5S)-2-(3,5-bis(trifluoromethyl)benzoyl)-5-(2-chloro-5-(trifluoromethyl)phenyl)pyrrolidine-1-carboxylate (5.00 g, 8.47 mmol) in toluene (20.0 mL) was added slowly. The resulting solution was stirred at room temperature for 2.5 h. The reaction was quenched by the addition of saturated NH₄Cl solution (40.0 mL). It was diluted with ethyl acetate (50 mL), and the layers were separated. The organic layer was washed with water, dried over anhydrous Na₂SO₄ and concentrated to yield tert-butyl (2S,5S)-2-((Z)-1-(3,5-bis(trifluoromethyl)phenyl)-3-ethoxy-3-oxoprop-1-en-1-yl)-5-(2-chloro-5-(trifluoromethyl)phenyl)pyrrolidine-1-carboxylate as a solid that was carried forward to the next step without further purification.
LC-MS APCI calc'd. for C₂₉H₂₇ClF₉NO₄ [M-Boc]⁺ 559.9, found 560.0.

**Step 2:** To a nitrogen purged solution of tert-butyl (2S,5S)-2-((Z)-1-(3,5-bis(trifluoromethyl)phenyl)-3-ethoxy-3-oxoprop-1-en-1-yl)-5-(2-chloro-5-(trifluoromethyl)phenyl)pyrrolidine-1-carboxylate (2.6 g, 3.93 mmol) in ethyl acetate (30.0 mL) was added platinum(IV) oxide (600 mg) and the resulting mixture was stirred at room temperature under H₂ for 16 h. The reaction mixture was filtered through a bed of diatomaceous earth and the filtrate was concentrated to yield tert-butyl (2S,5S)-2-((R)-1-(3,5-bis(trifluoromethyl)phenyl)-3-ethoxy-3-oxopropyl)-5-(2-chloro-5-(trifluoromethyl)phenyl)pyrrolidine-1-carboxylate as a solid that was carried forward to the next step without further purification.
LC-MS APCI calc'd. for C₂₉H₂₉CIF₉N0₄ [M + H -Boc]⁺ 562.2, found 562.2.

**Steps 3:** To a solution of tert-butyl (2S,5S)-2-((R)-1-(3,5-bis(trifluoromethyl)Dhenyl)-3-ethoxy-3-oxopropvyl)-5-(2-chloro-5-(trifluoromethyl)phenyl)pyrrolidine-1-carboxylate (2.3 g, 3.47 mmol) in dichloromethane (10.0 mL) was added trifluoroacetic acid (20.0 mL). The resulting solution was stirred at room temperature for 16 h. The reaction solution was concentrated. The residue was partitioned between ethyl acetate (100.0 mL) and aqueous 10% NaHCO₃ solution (50.0 mL). The organic layer was washed with water, dried over anhydrous Na₂SO₄ and concentrated to yield ethyl (R)-3-(3,5-bis(trifluoromethyl)phenyl)-3-((2S,5S)-5-(2-chloro-5-(trifluoromethyl)phenyl)pyrrolidin-2-yl)propanoate. The crude product was taken up in toluene (25.0 mL) in a 100 mL seal tube. Triethylamine (1.93 mL,14.9 mmol) was added, and the reaction solution was heated at100 °C for 16 h. The reaction solution was concentrated. The residue was diluted with ethyl acetate (75.0 mL), washed with water, dried over anhydrous Na₂SO₄ and concentrated. The crude product was purified by flash column chormatography to yield (1R,5S,7aS)-1-(3,5-bis(trifluoromethyl)phenyl)-5-(2-chloro-5-(trifluoromethyl)phenyl)hexahydro-3H-pyrrolizin-3-one as a solid.
LC-MS APCI calc'd. for C₂₂H₁₅ClF₉NO [M+H]⁺ 515.6, found 516.0.
¹H NMR (300 MHz, DMSO-d₆): δ 8.02 (s, 3H), 7.73-7.65 (m, 3H), 5.04 (t, J = 8.07 Hz, 1H), 4.63-4.55 (m, 1H), 4.30 (q, J = 7.77 Hz, 1H), 3.19-3.10 (m, 1H), 2.85 (q, J = 6.87 Hz, 1H), 2.63-2.56 (m, 1H), 1.60-1.54 (m, 1H), 1.37-1.33 (m, 1H), 1.15-1.00 (m, 1H).

The following intermediates (Table 1) were prepared according to Schemes A1 and B1 using the procedures outlined in the synthesis of intermediates A1 and B1.

**Table 1**

| **Lot** | **Structure** | **Name** | **Exact Mass [M+H]⁺** |
|---|---|---|---|
| **A2** | 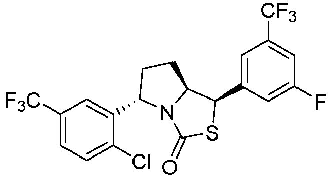 | (1R,5S,7aS)-5-(2-chloro-5-(trifluoromethyl)phenyl)-1-(3 -fluoro-5-(trifluoromethyl)phenyl)tetrahydro-1H,3H-pyrrolo[1,2-c]thiazol-3-one | Calc'd 484.03, Found 484.7 |
| **A3** | 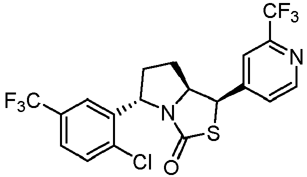 | (1R,5S,7aS)-5-(2-chloro-5-(trifluoromethyl)phenyl)-1-(2-(trifluoromethyl)pyridin-4-yl)tetrahydro-1H,3H-pyrrolo[1,2-c]thiazol- 3 -one | Calc'd 465.03, Found 465.0 |
| **B2** | 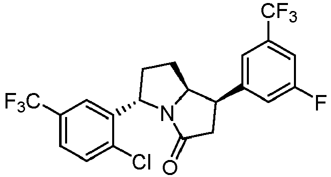 | (1R,5S,7aS)-5-(2-chloro-5-(trifluoromethyl)phenyl)-1-(3 -fluoro-5-(trifluoromethyl)phenyl)hexahydro-3H-pyrrolizin-3-one | Calc'd 466.07, Found 466.0 |
| **B3** | 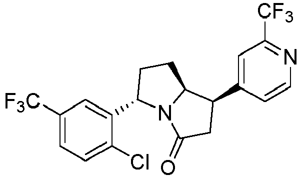 | (1R,5S,7aS)-5-(2-chloro-5-(trifluoromethyl)phenyl)-1-(2-(trifluoromethyl)pyridin-4-yl)hexahydro-3H-pyrrolizin-3-one | Calc'd 447.08, Found 447.2 |

### General Synthetic Schemes

Compounds of the present invention can be synthesized according to the general schemes outlined below. Syntheses of representative examples follow.

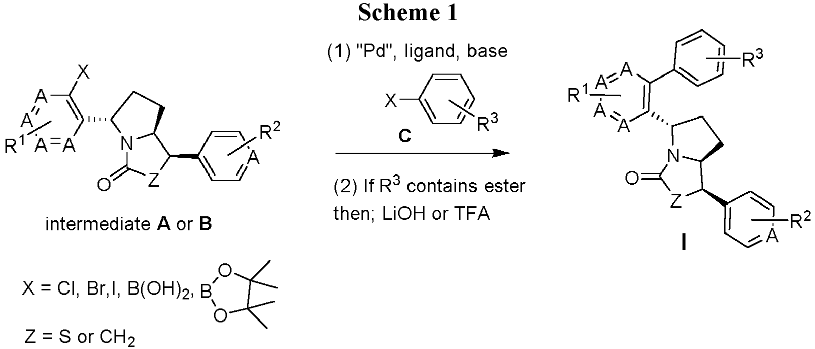

In accordance with Scheme **1**, a cross-coupling reaction between Intermediate **A** or **B** and an appropriately functionalized boronic acid/ester/halide **C** provides compounds of the general formula (**I**). Some of the intermediates **C** were synthesized according to published procedures in WO 2012058187, WO 2013063217 and WO 2013063217. In cases where an ester group is present in the product as a protecting group for the carboxylic acid, a saponification or deprotection step may subsequently be carried out to generate the acid.

### Example 1

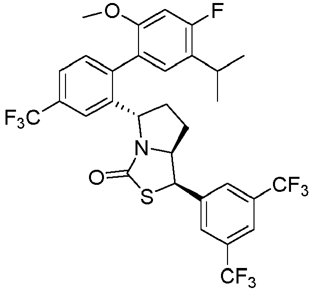

### (1R,5S,7aS)-1-(3,5-bis(trifluoromethyl)phenyl)-5-(4'-fluoro-5'-isopropyl-2'-methoxy-4-(trifluoromethyl)-[1,1'-biphenyl]-2-yl)tetrahydropyrrolo[1,2-c]thiazol-3(1H)-one (not part of the invention)

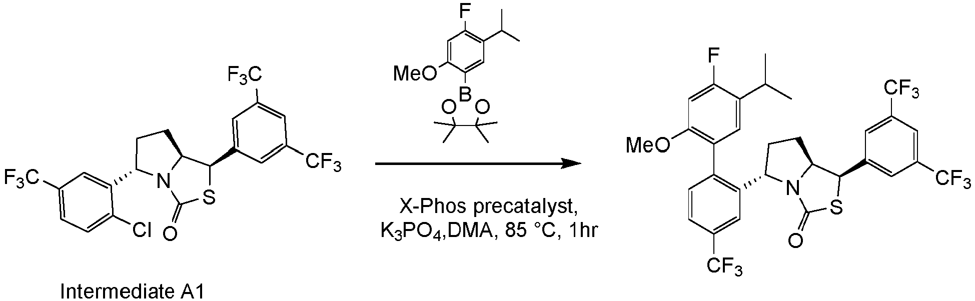

**Step 1:** In a microwave vial was taken (1R,5S,7aS)-1-(3,5-bis(trifluoromethyl)phenyl)-5-(2-chloro-5-(trifluoromethyl)phenyl)tetrahydropyrrolo[1,2-c]thiazol-3(1H)-one (20 mg, 0.037 mmol) in N,N-dimethylacetamide (0.5 mL). The mixture was degassed with nitrogen. Chloro(2-dicyclohexylphosphino-2',4',6'-triisopropyl-1,1'-biphenyl)[2-(2'-amino-1,1'-biphenyl)]palladium(II) (2.95 mg, 3.75 µmol), aqueous K₃PO₄ solution (0.037 mL, 0.075 mmol) were added, followed by (4-fluoro-5-isopropyl-2-methoxyphenyl)boronic acid (11.92 mg, 0.056 mmol). The resulting mixture was stirred at 85 °C for 1 hour. After cooling, EtOAc was added. The resulting mixture was washed sequentially with NH₄Cl solution and brine, dried over Na₂SO₄, filtered and concentrated. The crude product was purified by reverse phase HPLC to yield the title compound. ¹H NMR (500 MHz, CDCl₃, 1:1 mixture of atropisomers): δ 7.81-7.84 (m, 6 H, contains for two atropisomers protons); 7.53-7.56 (m, 4 H, contains for two atropisomers protons); 7.28 (s, 2H, contains for two atropisomers protons); 7.20 (d, J = 8.6 Hz, 1 H); 6.88 (d, J = 8.6 Hz, 1 H); 6.65 (dd, J = 23.7, 12.0 Hz, 2 H, contains for two atropisomers protons); 5.22 (d, J = 8.3 Hz, 1 H); 5.12-5.14 (m, 1 H); 5.02 (t, J = 8.1 Hz, 1 H); 4.84 (t, J = 8.1 Hz, 1 H); 4.73-4.78 (m, 1 H); 4.58-4.63 (m, 1 H); 3.80 (s, 3 H); 3.65 (d, J = 0.6 Hz, 3 H); 3.14-3.24 (m, 2 H); 1.17-1.29 (m, 12 H). MS ESI/APCI calc'd. for C₃₁H₂₅F₁₀NO₂S [M + H]+ 665.6, found 666.0. RTA (95% HS): 2350 nM

### Example 2

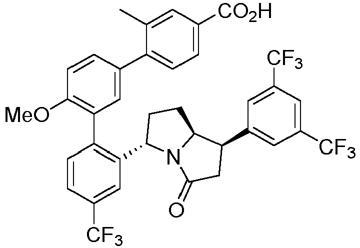

### 2"-((3S,7R,7aS)-7-(3,5-bis(trifluoromethyl)phenyl)-5-oxohexahydro-1H-pyrrolizin-3-yl)-4'-methoxy-2-methyl-4"-(trifluoromethyl)-[1,1':3',1"-terphenyl]-4-carboxylic acid

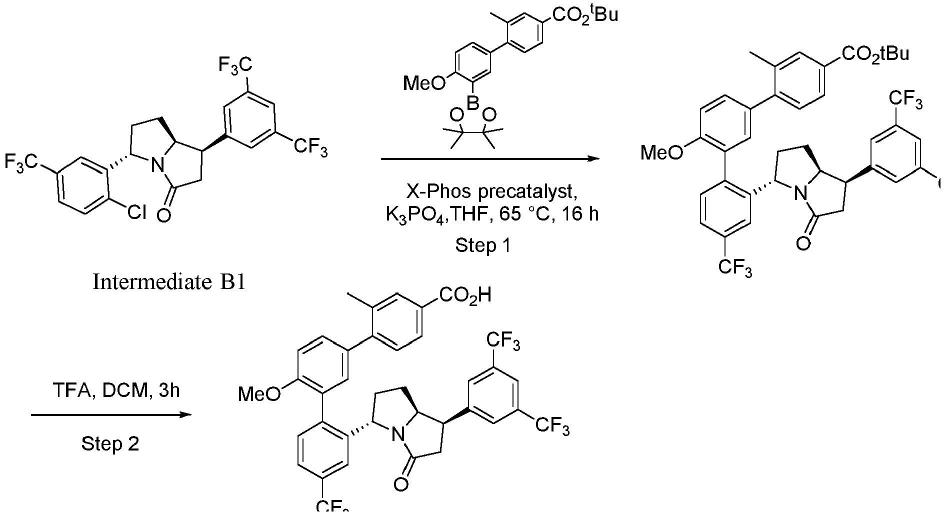

**Steps 1 and 2:** To intermediate B1 (0.050 g, 0.10 mmol) was added THF (3.0 mL), water (1.0 mL), tribasic potassium phosphate (0.053 g, 0.25 mmol), (4'-(tert-butoxycarbonyl)-4-methoxy-2'-methyl-[1,1'-biphenyl]-3-yl)boronic acid (0.051 g, 0.15 mmol) and chloro(2-dicyclohexylphosphino-2',4',6'-triisopropyl-1,1'-biphenyl)[2-(2-aminoethyl)phenyl)]palladium(II) (0.004 g, 0.005 mmol). The system was flushed with nitrogen gas and was heated at 65 °C for 16 h. The reaction was partitioned between ethyl acetate:hexanes (1:2, 10 mL) and water (10 mL). The organic layer was dried over sodium sulfate, filtered and concentrated. To the crude product dissolved in dichloromethane (2.0 mL), was added trifluoroacetic acid (0.2 mL). The mixture was stirred at room temperature for 3 h. TLC analysis indicated complete consumption of the starting material. The volatiles were removed under reduced pressure and the crude product was purified by reverse phase HPLC to yield 2"-((3S,7R,7aS)-7-(3,5-bis(trifluoromethyl)phenyl)-5-oxohexahydro-1H-pyrrolizin-3-yl)-4'-methoxy-2-methyl-4"-(trifluoromethyl)-[1,1':3',1"-terphenyl]-4-carboxylic acid as a solid. LC-MS APCI calc'd. for C₃₇H₂₈F₉NO₄ [M+H]⁺ 721.6, found 722.0.
¹H-NMR (400 MHz, DMSO-d₆, 80 °C): δ 12.88 (bs, 1H), 7.88-7.86 (m, 4H), 7.79 (d, J = 7.84 Hz, 1H), 7.66-7.61 (m, 2H), 7.42-7.38 (m, 2H), 7.34-7.33 (m, 1H), 7.20-7.22 (m, 2H), 4.90-4.80 (m, 1H), 4.54-4.46 (m, 1H), 4.20 (q, J = 8.36 Hz, 1H), 3.78 (s, 3H), 3.13-3.06 (m, 1H), 2.71-2.66 (m, 2H), 2.32 (s, 3H), 1.90-1.60 (m, 1H), 1.36-1.28 (m, 1H), 0.91-0.86 (m, 1H). RTA (95% HS): 1830 nM

'The following compounds in Table 2 were prepared according to general Scheme 1 using the procedure outlined in Examples 1 and 2 utilizing commercially available or known halide or boronic acids/esters.

**Table 2**

| **Ex** | **Structure** | **Name** | **IC₅₀ nM** | **MS (M+1)** |
|---|---|---|---|---|
| 3 | 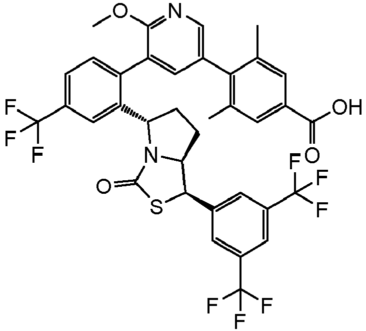 | 4-{5-[2-{(1R,5S,7aS)-1-[3,5-bis(trifluoromethyl)pheny l] -3-oxotetrahydro-1H-pyrrolo[1,2-c] [1,3]thiazol-5-yl}-4-(trifluoromethyl)phenyl] -6-methoxypyridin-3-yl}-3,5-dimethylbenzoic acid | 164 | Calc'd 755.16, found 755.13 |
| 4 | 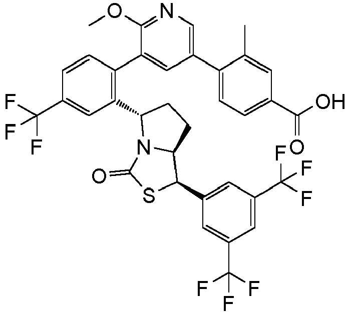 | 4-{5-[2-{(1R,5S,7aS)-1-[3,5-bis(trifluoromethyl)phenyl] -3-oxotetrahydro-1H-pyrrolo[1,2-c][1,3]thiazol-5-yl}-4-(trifluoromethyl)phenyl] -6-methoxypyridin-3-yl}-3-methylbenzoic acid | 559 | Calc'd 741.15, found 741.14 |
| 5 | 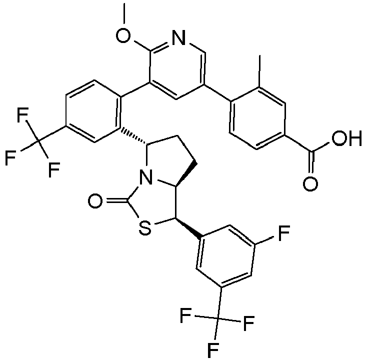 | 4-{5-[2-{(1R,5S,7aS)-1-[3-fluoro-5-(trifluoromethyl)phenyl] -3 - oxotetrahydro-1H-pyrrolo[1,2-c] [1,3]thiazol-5-yl}-4-(trifluoromethyl)phenyl] -6-methoxypyridin-3-yl}-3-methylbenzoic acid | 285 | Calc'd 691.15, found 692.1 |
| 6 | 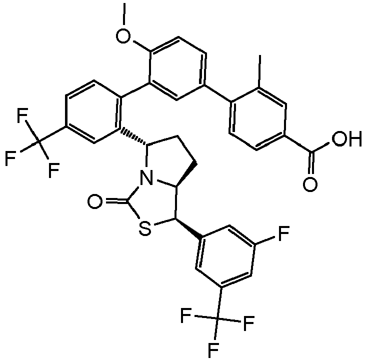 | 2"-{(1R,5S,7aS)-1-[3-fluoro-5-(trifluoromethyl)phenyl] -3 - oxotetrahydro-1H-pyrrolo[1,2-c] [1,3]thiazol-5-yl} -4'-methoxy-2-methyl-4" -(trifluoromethyl)-1,1':3',1"-terphenyl-4-carboxylic acid | 926 | Calc'd 690.15, found 691.1 |
| 7 | 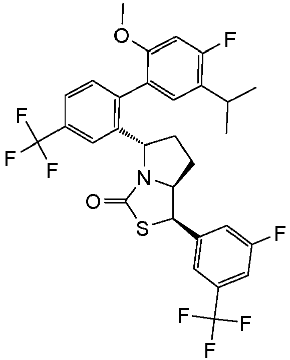 | (1R,5S,7aS)-5-[4'-fluoro-2'-methoxy-5'-(1-methylethyl)-4-(trifluoromethyl)biphenyl-2-yl]-1-[3-fluoro-5-(trifluoromethyl)phenyl]tetr ahydro-1H-pyrrolo[1,2-c] [1,3]thiazol-3-one | 1995 | Calc'd 616.16, found 617.1 |
| 8 | 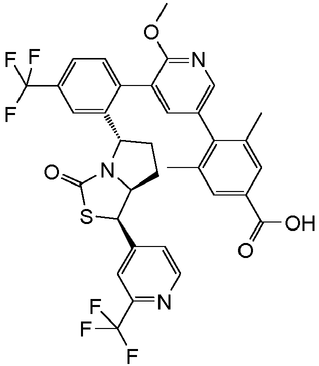 | 4-{6-methoxy-5-[2-{(1R,5S,7aS)-3-oxo-1-[2-(trifluoromethyl)pyridin-4-yl]tetrahydro-1H-pyrrolo[1,2-c] [1,3]thiazol-5-yl}-4-(trifluoromethyl)phenyl]pyr idin-3-yl}-3,5-dimethylbenzoic acid | 143 | Calc'd 688.17, found 689.1 |
| 9 | 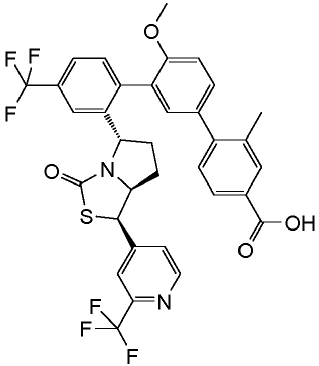 | 4'-methoxy-2-methyl-2"-{(1R,5S,7aS)-3-oxo-1-[2-(trifluoromethyl)pyridin-4-yl]tetrahydro-1H-pyrrolo[1,2-c] [1,3]thiazol-5-yl}-4"-(trifluoromethyl)-1,1':3',1"-terphenyl-4-carboxylic acid | 1992 | Calc'd 673.16, found 674.1 |
| 10 | 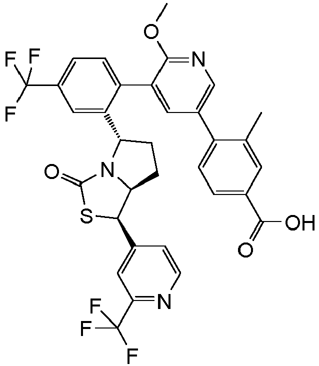 | 4-{6-methoxy-5-[2-{(1R,5S,7aS)-3-oxo-1-[2-(trifluoromethyl)pyridin -4-yl]tetrahydro-1H-pyrrolo[1,2-c] [1,3]thiazol-5-yl}-4-(trifluoromethyl)phenyl]pyr idin-3 -yl} -3 -methylbenzoic acid | 3945 | Calc'd 674.15, found 675.1 |
| 11 | 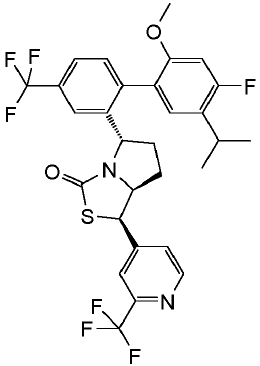 | (1R,5S,7aS)-5-[4'-fluoro-2'-methoxy-5'-(1-methylethyl)-4-(trifluoromethyl)biphenyl-2-yl]-1-[2-(trifluoromethyl)pyridin-4-yl]tetrahydro-1H-pyrrolo[1,2-c][1,3]thiazol-3-one | 2524 | Calc'd 599.16, found 600.1 |
| 12 | 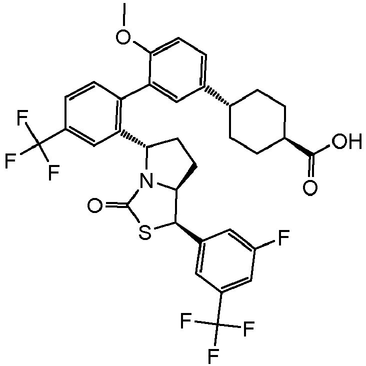 | trans-4-[2'-{(1R,SS,7aS)-1-[3-fluoro-5-(trifluoromethyl)phenyl]-3-oxotetrahydro-1H-pyrrolo[1,2-c][1,3]thiazol-5-yl}-6-methoxy-4'-(trifluoromethyl)biphenyl-3-yl]cyclohexanecarboxylic acid | 1503 | Calc'd 682.19, found 683.1 |
| 13 | 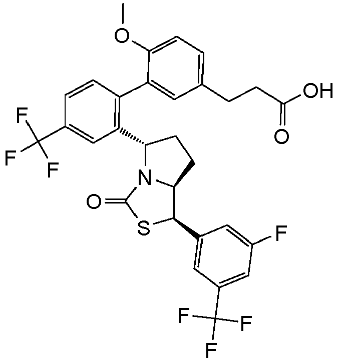 | 3-[2'-{(1R,SS,7aS)-1-[3-fluoro-5-(trifluoromethyl)phenyl]-3-oxotetrahydro-1H-pyrrolo[1,2-c][1,3]thiazol-5-yl}-6-methoxy-4'-(trifluoromethyl)biphenyl-3-yl]propanoic acid | 1977 | Calc'd 628.14, found 629.1 |
| 14 | 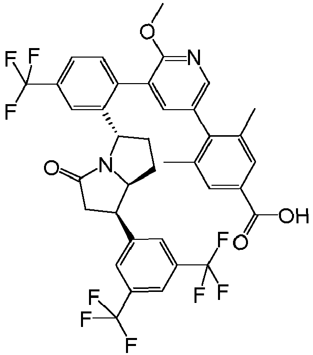 | 4-{5-[2-{(3S,7R,7aS)-7-[3,5-bis(trifluoromethyl)phenyl] -5-oxohexahydro-1H-pyrrolizin-3-yl}-4-(trifluoromethyl)phenyl]-6-methoxypyridin-3-yl}-3,5-dimethylbenzoic acid | 321 | Calc'd 737.21, found 738.2 |
| 15 | 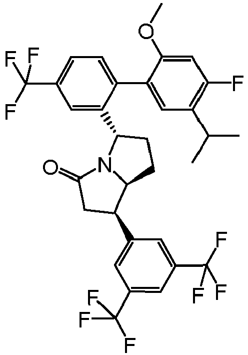 | (1R,5S,7aS)-1-[3,5-bis(trifluoromethyl)phenyl] -5-[4'-fluoro-2'-methoxy-5'-(1-methylethyl)-4-(trifluoromethyl)biphenyl-2-yl]hexahydro-3H-pyrrolizin-3-one | 2738 | Calc'd 648.2, found 649.2 |
| 16 | 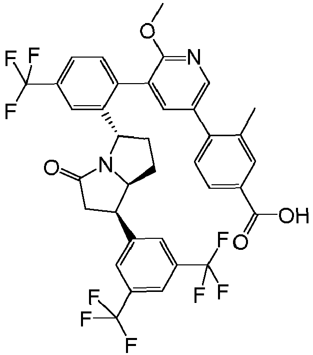 | 4-{5-[2-{(3S,7R,7aS)-7-[3,5-bis(trifluoromethyl)phenyl] -5-oxohexahydro-1H-pyrrolizin-3-yl}-4-(trifluoromethyl)phenyl]-6-methoxypyridin-3-yl} -3 - methylbenzoic acid | 2942 | Calc'd 723.19, found 724.1 |
| 17 | 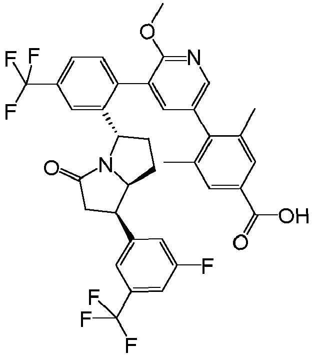 | 4-{5-[2-{(3S,7R,7aS)-7-[3-fluoro-5-(trifluoromethyl)phenyl]-5-oxohexahydro-1H-pyrrolizin-3-yl}-4-(trifluoromethyl)phenyl]-6-methoxypyridin-3-yl}-3,5-dimethylbenzoic acid | 328 | Calc'd 687.21, found 687.2 |
| 18 | 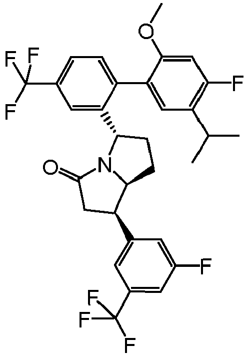 | (1R,5S,7aS)-5-[4'-fluoro-2'-methoxy-5'-(1-methylethyl)-4-(trifluoromethyl)biphenyl-2-yl]-1-[3-fluoro-5-(trifluoromethyl)phenyl]he xahydro-3H-pyrrolizin-3-one | 2694 | Calc'd 598.2, found 599.2 |
| 19 | 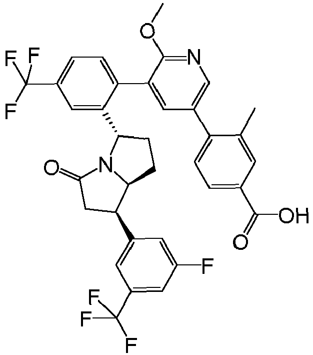 | 4-{5-[2-{(3S,7R,7aS)-7-[3-fluoro-5-(trifluoromethyl)phenyl]-5-oxohexahydro-1H-pyrrolizin-3-yl}-4-(trifluoromethyl)phenyl] -6-methoxypyridin-3-yl} -3 - methylbenzoic acid | 2879 | Calc'd 673.19, found 674.1 |
| 20 | 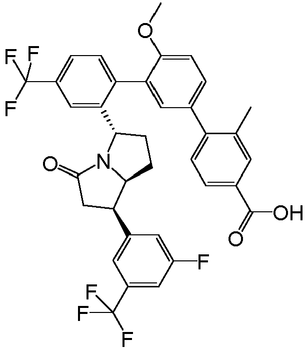 | 2"-{(3S,7R,7aS)-7-[3-fluoro-5-(trifluoromethyl)phenyl]-5-oxohexahydro-1H-pyrrolizin-3-yl}-4'-methoxy-2-methyl-4"-(trifluoromethyl)-1,1':3',1"-terphenyl-4-carboxylic acid | 4726 | Calc'd 672.2, found 673.2 |
| 21 | 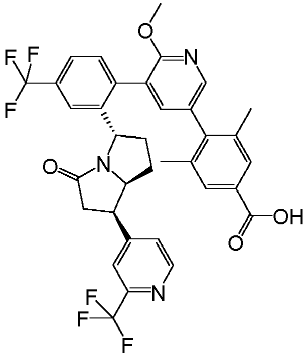 | 4-{6-methoxy-5-[2-{(3S,7R,7aS)-5-oxo-7-[2-(trifluoromethyl)pyridin-4-yl]hexahydro-1H-pyrrolizin-3-yl}-4-(trifluoromethyl)phenyl]pyr idin-3-yl}-3,5-dimethylbenzoic acid | 2899 | Calc'd 670.21, found 671.2 |

## Claims

1. A compound of Formula 1a, or a pharmaceutically acceptable salt thereof: 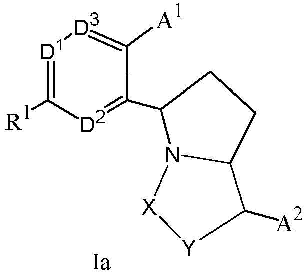
wherein X is -C(=O)-;
Y is CH₂;
R1 is CF₃;
D¹, D², and D³ are CH;
A¹ is phenyl or pyridyl, wherein A¹ is optionally substituted with 1-3 groups which are each independently F, -OCH₃, or isopropyl, and optionally one substituent group Z;
Z is A³ or -CH₂CH₂CO₂R⁸;
R⁸ is H or -CH₃;
A³ is phenyl, cyclohexyl, or pyridyl, wherein A³ is optionally substituted with 1-2 groups -CH₃ and is optionally substituted with 1 group -CO₂R⁸; and
A² is phenyl or pyridinyl, wherein A² is substituted with 1-2 groups which are selected from CF₃ and F.

2. The compound of claim 1, or a pharmaceutically acceptable salt thereof, having the structure below: 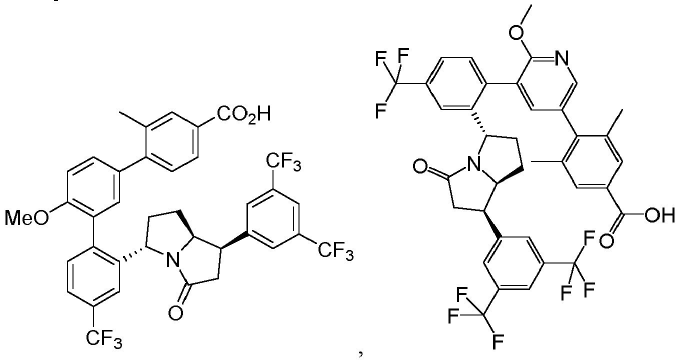 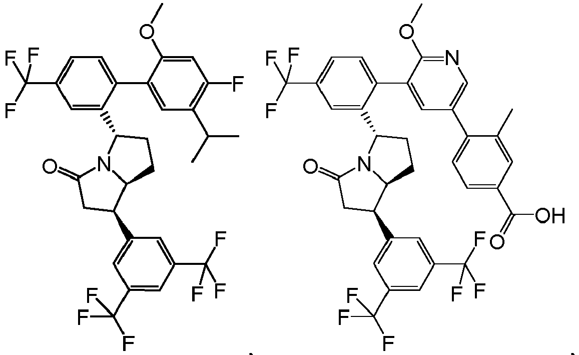 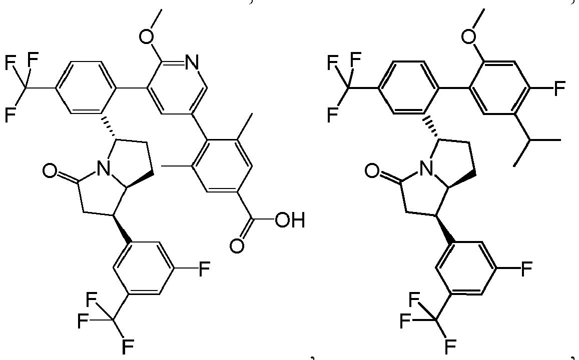 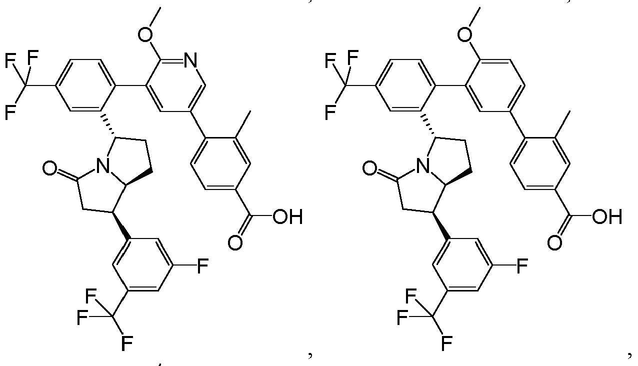 or

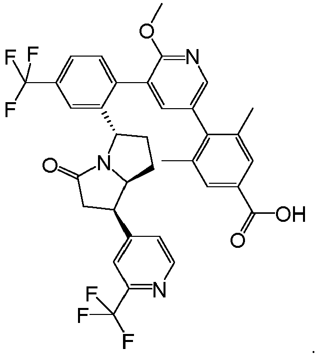

3. A pharmaceutical composition comprising a therapeutically effective amount of the compound of claim 1 or 2, or a pharmaceutically acceptable salt thereof, and a pharmaceutically acceptable carrier.

4. The compound of claim 1 or 2, or a pharmaceutically acceptable salt thereof, for use in any one selected from
a) a method of treating atherosclerosis
b) a method of raising HDL-C; or
c) a method of lowering LDL
d) a method of treating dyslipidemia

5. A pharmaceutical composition comprising the compound of claim 1 or 2, or a pharmaceutically acceptable salt thereof, a pharmaceutically acceptable carrier, and an additional active ingredient selected from:
(i) an HMG-CoA reductase inhibitor;
(ii) a bile acid sequestrant;
(iii) niacin and a related compound;
(iv) a PPARα agonist;
(v) a cholesterol absorption inhibitor;
(vi) an acyl CoA:cholesterol acyltransferase (ACAT) inhibitor;
(vii) a phenolic anti-oxidant;
(viii) a microsomal triglyceride transfer protein (MTP)/ApoB secretion inhibitor;
(ix) an anti-oxidant vitamin;
(x) a thyromimetic;
(xi) a LDL (low density lipoprotein) receptor inducer;
(xii) a platelet aggregation inhibitor;
(xiii) vitamin B 12 (also known as cyanocobalamin);
(xiv) folic acid or a pharmaceutically acceptable salt or ester thereof;
(xv) an FXR or LXR ligand;
(xvi) an agent that enhances ABCA1 gene expression;
(xvii) an ileal bile acid transporter; or
(xviii) a niacin receptor agonist.

## Patentansprüche

1. Eine Verbindung der Formel 1a oder ein pharmazeutisch annehmbares Salz davon: 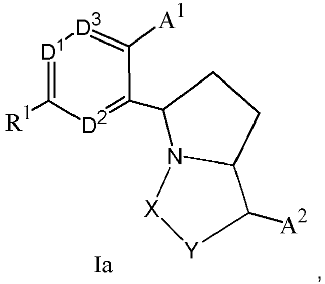
wobei X -C(=O)- ist,
Y CH₂ ist,
R¹ CF₃ ist,
D¹, D² und D³ CH sind,
A¹ Phenyl oder Pyridyl ist, wobei A1 gegebenenfalls substituiert ist mit 1-3 Gruppen, die jeweils unabhängig F, -OCH₃, oder Isopropyl sind, und gegebenenfalls einer Substituentengruppe Z,
Z A³ oder -CH₂CH₂CO₂R⁸ ist,
R⁸ H oder -CH₃ ist,
A³ Phenyl, Cyclohexyl oder Pyridyl ist, wobei A³ gegebenenfalls substituiert ist mit 1-2 Gruppen -CH₃ und gegebenenfalls substituiert ist mit 1 Gruppe -CO₂R⁸, und
A² Phenyl oder Pyridinyl ist, wobei A² substituiert ist mit 1-2 Gruppen, die ausgewählt sind aus CF₃ und F.

2. Die Verbindung nach Anspruch 1 oder ein pharmazeutisch annehmbares Salz davon, unten angegebene Struktur besitzend: 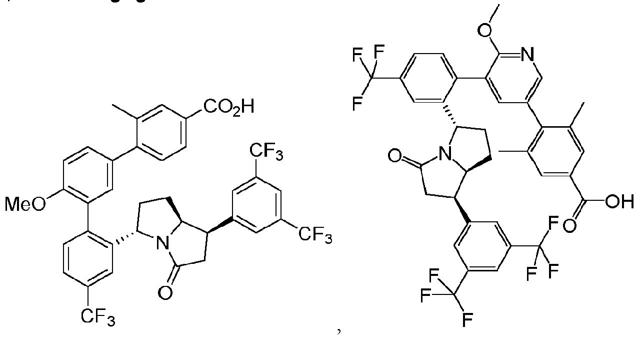 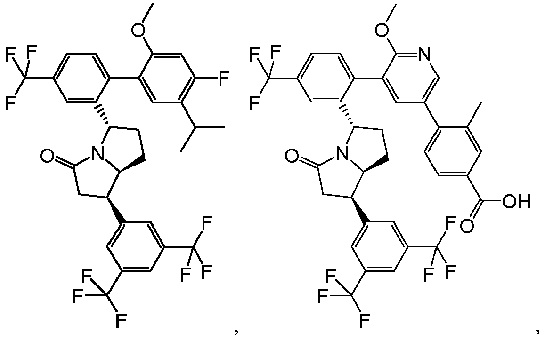 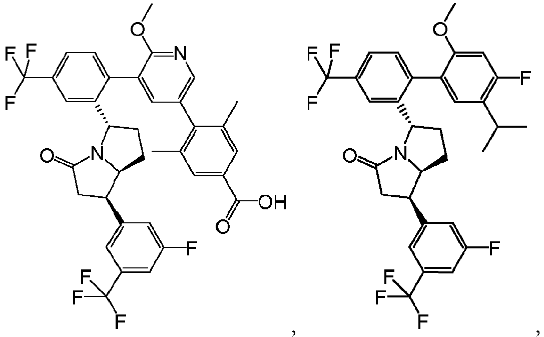 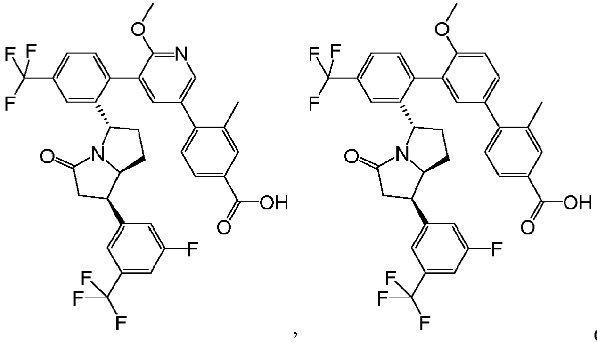 oder

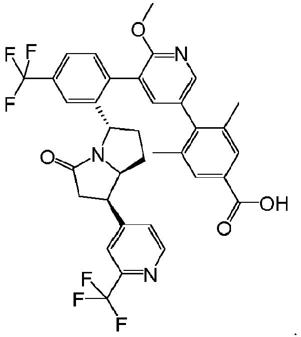

3. Eine pharmazeutische Zusammensetzung, umfassend eine therapeutisch wirksame Menge der Verbindung nach Anspruch 1 oder 2 oder eines pharmazeutisch annehmbaren Salzes davon, und einen pharmazeutisch annehmbaren Träger.

4. Die Verbindung nach Anspruch 1 oder 2 oder ein pharmazeutisch annehmbares Salz davon, zur Verwendung bei einem Verfahren, ausgewählt aus:
a) einem Verfahren zur Behandlung von Atherosklerose
b) einem Verfahren zur Erhöhung von HDL-C, oder
c) einem Verfahren zur Senkung von LDL
d) ein Verfahren zur Behandlung von Dyslipidämie.

5. Eine pharmazeutische Zusammensetzung, umfassend die Verbindung nach Anspruch 1 oder 2 oder ein pharmazeutisch annehmbares Salz davon, einen pharmazeutisch annehmbaren Träger und einen zusätzlichen Wirkstoff, ausgewählt aus:
(i) ein HMG-CoA-Reduktase-lnhibitor,
(ii) ein Gallensäuresequestriermittel,
(iii) Niacin und eine verwandte Verbindung,
(iv) ein PPARα-Agonist,
(v) ein Cholesterinabsorptionsinhibitor,
(vi) ein Acyl-CoA:Cholesterinacyltransferase(ACAT)-Inhibitor,
(vii) ein phenolisches Antioxidans,
(viii) ein mikrosomales Triglycerid-Transferprotein (MTP)/ApoB-Sekretions- Inhibitor,
(ix) ein antioxidatives Vitamin,
(x) ein Thyromimetikum,
(xi) ein LDL (Low-Density-Lipoprotein)-Rezeptorinduktor,
(xii) ein Thrombozytenaggregationsinhibitor,
(xiii) Vitamin B12 (auch bekannt als Cyanocobalamin),
(xiv) Folsäure oder ein pharmazeutisch annehmbares Salz oder ein pharmazeutisch annehmbarer Ester davon,
(xv) ein FXR- oder LXR-Ligand,
(xvi) ein Mittel, das ABCA1-Gen-Expression steigert,
(xvii) ein Ileum-Gallensäure-Transporter oder
(xviii) ein Niacinrezeptor-Agonist.

## Revendications

1. Composé de Formule la, ou un sel pharmaceutiquement acceptable de celui-ci : 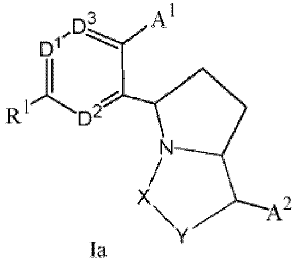
dans lequel X est -C(=O)- ;
Y est CH₂ ;
R¹ est CF₃ ;
D¹, D² et D³ sont CH;
A¹ est un phényle ou un pyridyle, dans lequel A¹ est optionnellement substitué par 1-3 groupes qui sont chacun indépendamment F, -OCH₃ ou un isopropyle et optionnellement un groupe substituant Z ;
Z est A³ ou -CH₂CH₂CO₂R⁸ ;
R⁸ est H ou -CH₃ ;
A³ est un phényle, un cyclohexyle ou un pyridyle, dans lequel A³ est optionnellement substitué par 1-2 groupes -CH₃ et il est optionnellement substitué par 1 groupe -CO₂R⁸ ; et
A² est un phényle ou un pyridyle, dans lequel A² est substitué avec 1-2 groupes qui sont choisis parmi CF₃ et F.

2. Composé selon la revendication 1, ou un sel pharmaceutiquement acceptable de celui-ci, présentant la structure ci-dessous : 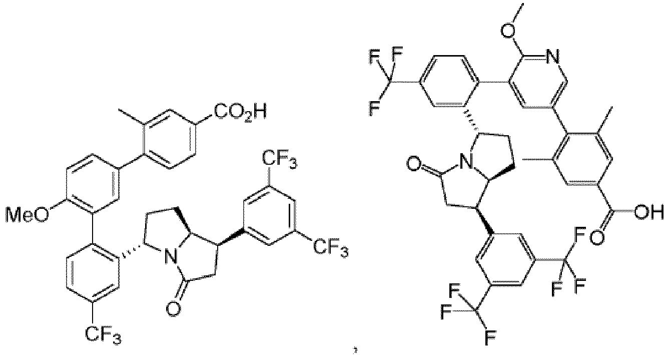 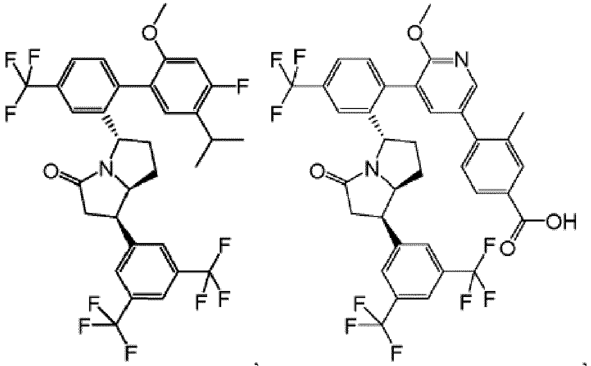 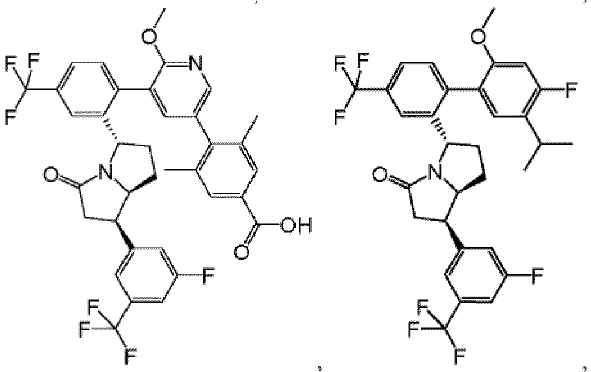 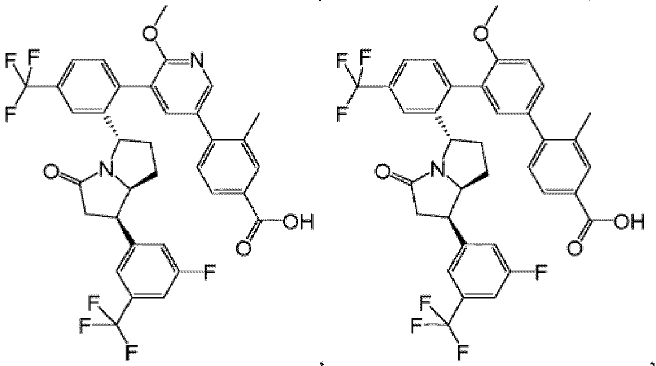 ou

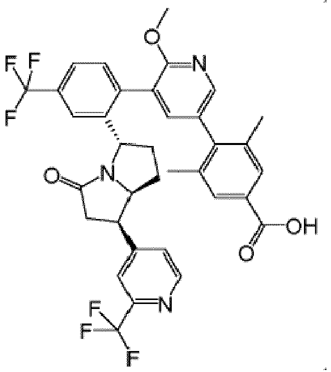

3. Composition pharmaceutique comprenant une quantité thérapeutiquement efficace du composé selon la revendication 1 ou 2, ou un sel pharmaceutiquement acceptable de celui-ci, et un véhicule pharmaceutiquement acceptable.

4. Composé selon la revendication 1 ou 2, ou un sel pharmaceutiquement acceptable de celui-ci, pour une utilisation dans l'un quelconque parmi :
a) un procédé de traitement d'une athérosclérose ;
b) un procédé d'augmentation de HDL-C ; ou
c) un procédé d'abaissement de LDL ;
d) un procédé de traitement d'une dyslipidémie.

5. Composition pharmaceutique comprenant le composé selon la revendication 1 ou 2, ou un sel pharmaceutiquement acceptable de celui-ci, un véhicule pharmaceutiquement acceptable et un ingrédient actif supplémentaire choisi parmi :
(i) un inhibiteur de HMG-CoA réductase ;
(ii) un chélateur des acides biliaires ;
(iii) la niacine et un composé apparenté ;
(iv) un agoni ste de PPARα ;
(v) un inhibiteur d'absorption de cholestérol ;
(vi) un inhibiteur d'acyl-CoA : cholestérol acyltransférase (ACAT) ;
(vii) un antioxydant phénolique ;
(viii) un inhibiteur de sécrétion de protéine de transfert de triglycéride microsomal (MTP)/ApoB ;
(ix) une vitamine antioxydante ;
(x) un thyromimétique ;
(xi) un inducteur de récepteur de LDL (lipoprotéine de basse densité) ;
(xii) un inhibiteur d'agrégation plaquettaire ;
(xiii) la vitamine B12 (aussi connue sous le nom de cyanocobalamine) ;
(xiv) l'acide folique ou un sel ou un ester pharmaceutiquement acceptables de celui-ci ;
(xv) un ligand de FXR ou LXR ;
(xvi) un agent qui augmente l'expression du gène ABCA1 ;
(xvii) un transporteur d'acide biliaire iléal ; ou
(xviii) un agoniste de récepteur de niacine.
